# EUROPEAN PATENT APPLICATION

(11) **EP 2 339 022 A2**
(43) Date of publication of application: **29.06.2011**
(21) Application number: 10196211.6
(22) Date of filing: 25.04.2008
(51) Int. Cl.: C12Q 1/68

(54) **Use of methylated or unmethylated line-1 DNA as a cancer marker**

(30) Priority: 25.04.2007 US 913880 P
(62) Divisional of application: 08769195.2
(71) Applicant: John Wayne Cancer Institute, Santa Monica, CA 90404 (US)
(72) Inventor: Hoon, Dave S.B., Los Angeles, CA 90025 (US); Sunami, Eiji, Santa Monica, CA 90404 (US)
(74) Representative: Viering, Hans-Martin

(57) **Abstract**

The invention relates to a method of detecting LINE-1 (long interspersed nucleotide elenients-I) DNA either methy-lated or unmethylated at the promoter region in a tissue or body fluid sample from a subject. Also disclosed are methods of using LINE-1 DNA as a biomarker for diagnosing, predicting, and monitoring cancer progression and treatment.

## Description

### RELATED APPLICATION

This application claims priority to U.S. Provisional Application Serial No. 60/913,880, filed on April 25, 2007, the content of which is incorporated herein by reference in its entirety.

### FIELD OF THE INVENTION

The present invention relates in general to the long interspersed nuclear elements (LINEs). More specifically, the invention relates to the use of unmethylated LINE-1 DNA as a diagnostic, prognostic, and predictive biomarker in the management of cancer.

### BACKGROUND OF THE INVENTION

Repetitive sequences are known as junk DNA and account for at least 50% of the human genome. About 90% of those human repetitive sequences belong to transposable elements. LINEs are one of the superfamilies of those transposon-derived repeats and account for 20% of the human genome. Three LINE families, LINE1, LINE2, and LINE3, are found in the human genome. Among those families, only LINE 1 is capable of transposition, is most abundant, and accounts for 17% of human DNA. The size of the full-length LINE1 is about 6.1 kb. Over 500,000 sequences exist in the entire human genome.

LINE1 contains a promoter sequence and two open reading frames (ORF1 and ORF2). ORF1 encodes an RNA binding protein; ORF2 encodes an endonuclease-reverse transcriptase protein. During retrotransposition, LINE1 is transcribed into RNA, reverse transcribed into cDNA, and reintegrated into the genome at a new site.

### SUMMARY OF THE INVENTION

The present invention is based, at least in part, upon the unexpected discovery that LINE-1 (long interspersed nucleotide elements-1) DNA can be detected in a body fluid and that LINE-1 either methylated or unmethylated at the promoter region can be used as a biomarker for diagnosis and prognosis of cancer.

Accordingly, in one aspect, the invention features a method of detecting LINE-1 DNA in a body fluid. The method comprises providing a body fluid sample from a subject and detecting LINE-1 DNA in the sample. In some embodiments, the method further comprises detecting methylation or unmethylation of the LINE-1 DNA at the promoter region.

In another aspect, the invention features a method of determining whether a subject is suffering from cancer. One method of the invention comprises providing a body fluid sample from a subject and determining the level of LINE-1 DNA in the sample. If the level of the LINE-1 DNA in the sample is higher than a control LINE-1 level in a normal sample, the subject is likely to be suffering from cancer.

Another method of determining whether a subject is suffering from cancer comprises providing from a subject a sample of a tissue where esophageal cancer, colorectal cancer, melanoma, or breast cancer may develop and determining the level of LINE-1 DNA in the sample. If the level of the LINE-1 DNA in the sample is higher than a control LINE-1 level in a normal sample, the subject is likely to be suffering from esophageal cancer, colorectal cancer, melanoma, or breast cancer.

Also within the invention is a method of monitoring cancer. The method comprises providing a tumor or body fluid sample from a subject suffering from cancer and determining the level of LINE-1 DNA in the sample. If the level of the LINE-1 DNA in the sample is higher than a control LINE-1 level in a control tumor or body fluid sample from a control subject suffering from the cancer, the cancer is likely to be at a more advanced stage in the subject than in the control subject, the subject is likely to be less responsive to a cancer therapy than the control subject, the subject is likely to have a decreased probability of survival than the control subject, or the tumor genetic instability is likely to be higher in the subject than in the control subject. On the other hand, if the level of the LINE-1 DNA in the sample is lower than a control LINE-1 level in a control tumor or body fluid sample from a control subject suffering from the cancer, the cancer is likely to be at a less advanced stage in the subject than in the control subject, the subject is likely to be more responsive to a cancer therapy than the control subject, the subject is likely to have an increased probability of survival than the control subject, or the tumor genetic instability is likely to be lower in the subject than in the control subject.

More specifically, if the level of the LINE-1 DNA in the sample is higher than the control LINE-1 level in the control tumor or body fluid sample from the control subject suffering from the cancer, the level of RASSF1a, RARb, GSTP1, or MGMT gene unmethylated at the promoter region is likely to be higher in the sample than in the control sample. If the level of the LINE-1 DNA in the sample is lower than the control LINE-1 level in the control tumor or body fluid sample from the control subject suffering from the cancer, the level of RASSF1a, RARb, GSTP1, or MGMT gene unmethylated at the promoter region is likely to be lower in the sample than in the control sample.

In prostate cancer, if the level of the LINE-1 DNA in the sample is higher than the control LINE-1 level in the control tumor or body fluid sample from a control subject suffering from a multifocal prostate cancer, the subject is likely to be suffering from a unifocal prostate cancer. If the level of the LINE-1 DNA in the sample is lower than the control LINE-1 level in the control tumor or body fluid sample from a control subject suffering from a unifocal prostate cancer, the subject is likely to be suffering from a multifocal prostate cancer.

In addition, if the level of the LINE-1 DNA in the sample is higher than the control LINE-1 level in the control tumor or body fluid sample from the control subject suffering from the cancer, the prostate volume is likely to be larger in the subject than in the control subject, or the PSA density is likely to be higher in the subject than in the control subject. If the level of the LINE-1 DNA in the sample is lower than the control LINE-1 level in the control tumor or body fluid sample from the control subject suffering from the cancer, the prostate volume is likely to be smaller in the subject than in the control subject, or the PSA density is likely to be lower in the subject than in the control subject.

LINE-1 DNA may exist as cellular or acellular DNA in a subject. A body fluid may be blood, serum, plasma, bone marrow, peritoneal fluid, or cerebral spinal fluid. In some embodiments, the subject suffers from cancer such as prostate cancer, esophageal cancer, colorectal cancer, melanoma, or breast cancer. The level of LINE-1 DNA may be represented by the level of the LINE-1 DNA either methylated or unmethylated at the promoter region, the level of the LINE-1 DNA unmethylated at the promoter region, or the ratio of the level of the LINE-1 DNA unmethylated at the promoter region to the level of the LINE-1 DNA either methylated or unmethylated at the promoter region.

Unless otherwise defined, all technical and scientific terms used herein have the same meaning as commonly understood by one of ordinary skill in the art to which this invention pertains. In case of conflict, the present document, including definitions, will control. The materials, methods, and examples disclosed herein are illustrative only and not intended to be limiting. Other features, objects, and advantages of the invention will be apparent from the description and the accompanying drawings, and from the claims.

### BRIEF DESCRIPTION OF THE FIGURES

**Figure 1****.** Prostate cancer study. LINE1 unmethylation index was calculated for each sample. The y-axis represents the LINE1 unmethylation index (the copy number of unmethylated LINE1 divided by those of unmethylated LINE1 plus methylated LINE1). Serum DNA from prostate cancer patients showed significantly higher LINE1 unmethylation index than those from normal human (n = 40 for normal human males and n = 73 for cancer patients. Average of LINE1 unmethylated index is 0.028 for normal human and 0.079 for cancer patients, respectively. P=0.0002).

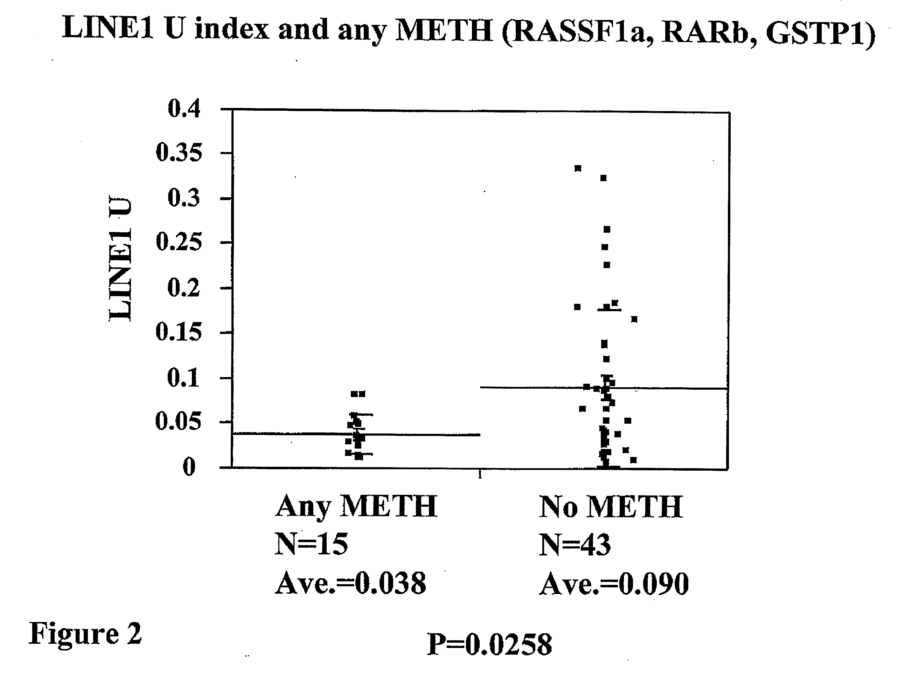

**Figure 2****.** Prostate cancer study. The relation between the LINE1 unmethylation index and methylation status of other tumor related genes. The y-axis represents the LINE1 unmethylation index (the copy number of unmethylated LINE1 divided by those of unmethylated LINE1 plus methylated LINE1). Cancer patients were divided into two groups, any methylation group and no methylation group, according to their status of three tumor-related genes (RASSF1a, RARb, GSTP1). No methylation group (n=15) showed higher LINE1 unmethylation index than any methylation group (n=43). LINE1 unmethylation index of serum DNA from prostate cancer patients correlates with the methylation status of other cancer-related genes (P=0.0258).
**Figure 3****.** Prostate cancer analysis of LINE1 circulating DNA in serum. LINE1 unmeth / unmeth+meth; LINE1 U index in serum DNA. Comparison of normal male donor serum versus AJCC stage I, II, III, and IV prostate cancer patients.
Figure 4. Prostate cancer analysis of LINE1 circulating DNA in serum. LINE1 unmeth / unmeth+meth; LINE1 U index in serum DNA ROC.
**Figure 5****.** Prostate cancer study of LINE 1 circulating DNA integrity in serum. LINE1 103; DNA volume in serum DNA. Comparison of normal male donor serum versus AJCC stage I, II, III, and IV prostate cancer patients.
**Figure 6**. Prostate cancer study of LINE1 circulating DNA integrity in serum.. LINE1 103; DNA volume in serum DNA. Normal male donors vs AJCC stage IV prostate cancer patients.
**Figure 7****.** Prostate cancer study of LINE1 circulating DNA integrity in serum. LINE1 103; DNA volume in serum DNA ROC.
**Figure 8****.** Prostate cancer analysis of LINE1 circulating DNA in serum. LINE1 unmeth copy number in serum DNA. Comparison of normal male donor serum versus AJCC stage I, II, III, and IV prostate cancer patients.
**Figure 9****.** Prostate cancer study; LINE1 unmethylated copy number in serum. LINE1 unmeth copy number in serum DNA. Comparison of normal male donors to AJCC stage IV patients.
**Figure 10****.** Prostate cancer study. LINE1 unmeth / unmeth+meth; LINE1 U index in serum DNA. Comparison of normal male donors to AJCC stage IV patients.
**Figure 11****.** Prostate cancer study. LINE1 unmeth copy number in serum DNA ROC.
**Figure 12****.** Prostate cancer study of serum circulating DNA. Gleason Score. vs LINE 1 U index, LINE103, and LINE1 U.
**Figure 13****.** Prostate cancer study of serum circulating DNA. PSA (cut off 4.0) vs LINE1 U index, LINE103, and LINE1 U.
**Figure 14****.** Prostate cancer study of serum circulating DNA. PSA (cut off 10.0) vs LINE1 U index, LINE 103, and LINE1 U.
**Figure 15****.** Prostate cancer study. A. Correlation between tumor unmethylation index (U index) and multifocality. Unifocal cancer showed significantly high U index compared with multifocal cancer (p=0.0067). BE. Correlation between tumor U index and clinicopathologic variables. There is no significant difference between tumor U index and clinicopathologic variables. F. Correlation between tumor U index and prostate volume. Tumor U index is significantly correlated with prostate volume (p=0.0191).
**Figure 16**. LINE-1 U index in esophageal squamous cell carcinoma. Comparison of adjacent normal epithelium to primary and lymph node metastasis.
**Figure 17****.** LINE-1 U index of each tumor depth in esophageal squamous cell carcinoma.
**Figure 18****.** LINE-1 U index (Unmeth / Unmeth + Meth) in esophageal squamous cell carcinoma ROC curves.
**Figure 19****.** LINE1 unmeth by AQAMA and OCSBM. Normal mucosa; comparison among normal human, colorectal adenoma patients, and colorectal cancer patients.
**Figure 20****.** LINE1 unmeth by AQAMA and OCSBM. Normal mucosa and adenoma; comparison between colorectal adenoma patients and colorectal cancer with adenoma patients.
**Figure 21****.** LINE1 unmeth by AQAMA and OCSBM. Colorectal cancer with colorectal adenoma patients; comparison among adjacent normal mucosa, colorectal adenoma, colorectal cancer, and colorectal cancer parenchyma.
**Figure 22****.** LINE1 unmeth by AQAMA and OCSBM. Comparison among normal colorectal mucosakeep in tissue, colorectal adenoma, early colorectal cancer, and advanced colorectal cancer.
**Figure 23****.** LINE1 unmeth by AQAMA and OCSBM. Comparison among colorectal normal mucosakeep in tissue, colorectal adenoma, early colorectal cancer, and advanced colorectal cancer.
**Figure 24****.** Laser capture microdissection of colorectal tissue separation from paraffin-embedded tissue section.
**Figure 25****.** Laser capture microdissection of colorectal tissue separation from paraffin-embedded tissue section.
**Figure 26****.** Laser capture microdissection of colorectal tissue separation from paraffin-embedded tissue section.
**Figure 27****.** Colorectal adenoma study. On cap SBM optimization; DNA volume.
**Figure 28****.** Colorectal adenoma study. On cap SBM optimization; conversion ratio.
**Figure 29****.** LINE-1 U index in melanoma tissue. Comparison of normal skin to primary or metastatic melanomas.
**Figure 30****.** LINE-1 U index in melanoma tissue. Comparison of normal skin, primary melanomas and metastatic melanomas.
**Figure 31****.** LINE-1 U index in melanoma tissue. Comparison of normal skin to different AJCC stages of primary and metastatic tumors.
**Figure 32****.** LINE1 copy number in serum and biochemotherapy treatment in melanoma patients. Comparison of poor and good responders in Stage IV melanoma patients.
**Figure 33****.** LINE1 copy number in serum and biochemotherapy treatment in Stage IV melanoma patients.
**Figure 34****.** Pico (double strand) and Oligo (single) vs LINE103 copy number in serum. Assessment of DNA integrity in melanoma patients serum.
**Figure 35****.** Melanoma metastasis vs. primary; LINE1 unmethylation. Significant difference; p < 0.05.
**Figure 36****.** Melanoma tumor metastasis vs. normal skin vs. primary tumor; LINE1 unmethylation. Significant difference; p < 0.05.
**Figure 37****.** Unmethylation index of LINE1 for breast cancer tissue.
**Figure 38****.** LINE1 copy number: normal vs. cancer (all stages).
**Figure 39****.** LINE1 copy number: normal vs. cancer.
**Figure 40****.** LINE1 copy number vs. AJCC stage (1,2,3).
**Figure 41****.** LINE1 copy number vs. AJCC stage (1,2,3).
**Figure 42****.** LINE1 copy number: T stage.
**Figure 43****.** LINE1 copy number: N stage.
**Figure 44****.** LINE1 copy number vs. AJCC stage (normal,0,1,2,3).
**Figure 45****.** LINE1 copy number vs. AJCC stage (0+1,2,3).
**Figure 46****.** LINE1 copy number vs. AJCC stage (1,2,3).
**Figure 47****.** Normal vs. cancer unmethylation status.
**Figure 48****.** Unmethylation status: normal vs. cancer (all stages); normal vs. stage I, II, III, IV; normal vs. stage II, III, IV; and normal vs. stage III, IV.
**Figure 49****.** Unmethylation status by AJCC stage (0,1,2,3).
**Figure 50****.** Unmethylation status by T stage (0,1,2,3,4).
**Figure 51****.** Unmethylation status by N stage (0,1,2,3).
**Figure 52****.** Unmethylation status by N stage (negative vs. positive).
**Figure 53****.** ER negative vs. positive; PR negative vs. positive; and HER2 negative vs. positive.

### DETAILED DESCRIPTION OF THE INVENTION

LINE1 contains CpG islands in its promoter region which are significantly methylated under normal conditions. In breast, melanoma, esophageal, colorectal, and prostate cancer, unmethylated LINE1 was found to be elevated. The level of unmethylated LINE1 is believed to be related to genetic instability of tumor cells and methylation status of its tumor related/suppressor genes. The inventors developed a quantitative assay using real-time PCR and AQAMA to assess methylated or unmethylated LINE1 a s circulating DNA in blood. Assessment of serum in prostate cancer, melanoma, and breast cancer patients demonstrated higher unmethylation index of LINE1 compared to respective normal control individuals. LINE1 methylation status was related to overall methylation status of tumor tissue. Circulating LINE1 methylation status can be used as a surrogate of tumor genetic instability (i.e., loss of heterozogozyity, epigenetic changes, translocation, etc). LINE1 methylation status can also be used to assess human melanoma.

LINE1 methylation can be used in combination with other circulating DNA biomarkers such as methylation, chromosome instability, mutation, chromosome translocation, and loss of heterozygosity of microsatellites for diagnosis, prognosis, and prediction in breast, melanoma, and prostate cancer.

The following represent embodiments of the present invention:
1. LINE1 methylation as a surrogate marker for tumor detection in blood.
2. Uncoding regions of genome as biomarkers in blood.
3. LINE1 methylation as a prognostic circulating DNA biomarker in body fluids for breast and prostate cancer.
4. Methylation status of LINE1 is predictive of tumor genetic instability. Detection in blood instead of actual tumor biopsy is an advantage.
5. Unmethylation index of LINE1 in blood (serum/plasma) for detection of cancer.
6. Unmethylation index of LINE1 in blood as a surrogate of tumor genetic instability without sampling tumor.
7. Repetitive monitoring of patients' blood for detection of genetic instability.
8. Assessment of LINE 1 status in blood before, during, and after treatment.

Accordingly, the invention first provides a method of detecting LINE-1 DNA in a body fluid. The term "body fluid" refers to any body fluid in which acellular DNA or cells (e.g., cancer cells) may be present, including, without limitation, blood, serum, plasma, bone marrow, cerebral spinal fluid, peritoneal/pleural fluid, lymph fluid, ascite, serous fluid, sputum, lacrimal fluid, stool, and urine. Body fluid samples can be obtained from a subject using any of the methods known in the art.

As used herein, a "subject" refers to a human or animal, including all mammals such as primates (particularly higher primates), sheep, dog, rodents (e.g., mouse or rat), guinea pig, goat, pig, cat, rabbit, and cow. In a preferred embodiment, the subject is a human. In another embodiment, the subject is an experimental animal or animal suitable as a disease model.

LINE-1 DNA may exist as either cellular or acellular DNA in a subject. "Acellular DNA" refers to DNA that exists outside a cell in a body fluid of a subject or the isolated form of such DNA. "Cellular DNA" refers to DNA that exists within a cell or is isolated from a cell.

Methods for extracting acellular DNA from body fluid samples are well known in the art. Commonly, acellular DNA in a body fluid sample is separated from cells, precipitated in alcohol, and dissolved in an aqueous solution. Methods for extracting cellular DNA from body fluid samples are also well known in the art. Typically, cells are lysed with detergents. After cell lysis, proteins are removed from DNA using various proteases. DNA is then extracted with phenol, precipitated in alcohol, and dissolved in an aqueous solution.

The presence of LINE-1 DNA is then detected in the body fluid sample. The genomic sequence of LINE-1 is known. The presence of the LINE-1 genomic sequence can be determined using many techniques well known in the art. Such techniques include, but are not limited to, Southern blot, sequencing, and PCR.

In some embodiments, the method further comprises detecting methylation or unmethylation of the LINE-1 DNA at the promoter region. A "promoter" is a region of DNA extending 150-300 bp upstream from the transcription start site that contains binding sites for RNA polymerase and a number of proteins that regulate the rate of transcription of the adjacent gene. The promoter region of LINE-1 is well known in the art. Methylation or unmethylation of the LINE-1 promoter can be assessed by any method commonly used in the art, for example, methylation-specific PCR (MSP), bisulfite sequencing, or pyrosequencing.

MSP is a technique whereby DNA is amplified by PCR dependent upon the methylation state of the DNA. See, e.g., U.S. Patent No. 6,017,704. Determination of the methylation state of a nucleic acid includes amplifying the nucleic acid by means of oligonucleotide primers that distinguish between methylated and unmethylated nucleic acids. MSP can rapidly assess the methylation status of virtually any group of CpG sites within a CpG island, independent of the use of methylation-sensitive restriction enzymes. This assay entails initial modification of DNA by sodium bisulfite, converting all unmethylated, but not methylated, cytosines to uracils, and subsequent amplification with primers specific for methylated versus unmethylated DNA. MSP requires only small quantities of DNA, is sensitive to 0.1% methylated alleles of a given CpG island locus, and can be performed on DNA extracted from body fluid samples. MSP eliminates the false positive results inherent to previous PCR-based approaches which relied on differential restriction enzyme cleavage to distinguish methylated from unmethylated DNA. This method is very simple and can be used on small amounts of samples. MSP product can be detected by gel electrophoresis, CAE (capillary array electrophoresis), or real-time quantitative PCR.

Bisulfite sequencing is widely used to detect 5-MeC (5-methylcytosine) in DNA, and provides a reliable way of detecting any methylated cytosine at single-molecule resolution in any sequence context. The process of bisulfite treatment exploits the different sensitivity of cytosine and 5-MeC to deamination by bisulfite under acidic conditions, in which cytosine undergoes conversion to uracil while 5-MeC remains unreactive.

In some embodiments, the subject suffers from cancer. As used herein, "cancer" refers to a disease or disorder characterized by uncontrolled division of cells and the ability of these cells to spread, either by direct growth into adjacent tissue through invasion, or by implantation into distant sites by metastasis. Exemplary cancers include, but are not limited to, carcinoma, adenoma, lymphoma, leukemia, sarcoma, mesothelioma, glioma, germinoma, choriocarcinoma, prostate cancer, lung cancer, breast cancer, colorectal cancer, gastrointestinal cancer, bladder cancer, pancreatic cancer, endometrial cancer, ovarian cancer, melanoma, brain cancer, testicular cancer, kidney cancer, skin cancer, thyroid cancer, head and neck cancer, liver cancer, esophageal cancer, gastric cancer, intestinal cancer, colon cancer, rectal cancer, myeloma, neuroblastoma, and retinoblastoma. Preferably, the cancer is prostate cancer, esophageal cancer, colorectal cancer, melanoma, or breast cancer.

The invention further provides a method of determining whether a subject is suffering from cancer. In one such method, a body fluid sample is obtained from a subject, and the level (e.g., copy number) of LINE-1 DNA in the sample is determined. If the level of the LINE-1 DNA in the sample is higher than a control LINE-1 level in a normal sample, the subject is likely to be suffering from cancer. A "normal sample" is a sample obtained from a normal subject.

The level of LINE-1 DNA may be represented by the level of the LINE-1 DNA either methylated or unmethylated at the promoter region (i.e., the sum of the level of the LINE-1 DNA methylated at the promoter region and the level of the LINE-1 DNA unmethylated at the promoter region), the level of the LINE-1 DNA unmethylated at the promoter region, the ratio of the level of the LINE-1 DNA unmethylated at the promoter region to the level of the LINE-1 DNA either methylated or unmethylated at the promoter region, or any other mathematical formula positively relating to the level of the LINE-1 DNA unmethylated at the promoter region.

In another method of determining whether a subject is suffering from cancer, a sample of a tissue where esophageal cancer, colorectal cancer, melanoma, or breast cancer may develop is obtained from a subject, and the level of LINE-1 DNA in the sample is determined. If the level of the LINE-1 DNA in the sample is higher than a control LINE-1 level in a normal sample, the subject is likely to be suffering from esophageal cancer, colorectal cancer, melanoma, or breast cancer.

Tissue samples can be obtained from a subject using any of the methods known in the art. The level of LINE-1 DNA in a tissue sample may be determined as described above. A "normal tissue sample" may be obtained from a normal subject or a normal tissue of a test subject. Preferably, the normal tissue is obtained from a site where the cancer being tested for can originate or metastasize.

The invention also provides methods of monitoring cancer progression and treatment, as well as methods for predicting the outcome of cancer. These methods involve obtaining a tumor or body fluid sample from a subject suffering from cancer, determining the level of LINE-1 DNA in the sample, and comparing it to a control LINE-1 level in a control tumor or body fluid sample from a control subject suffering from the cancer. A "control subject" may be a different subject suffering from the same type of cancer, or the same subject at a different time point, e.g., at a different cancer stage, or before, during, or after a cancer therapy (e.g., a surgery or chemotherapy).

If the level of the LINE-1 DNA in the test sample is higher than in the control sample, the cancer is likely to be at a more advanced stage in the test subject than in the control subject, the test subject is likely to be less responsive to a cancer therapy than the control subject, the test subject is likely to have a decreased probability of survival than the control subject, or the tumor genetic instability (e.g., epigenetic changes, methylation, chromosome instability, mutation, chromosome translocation, and loss of heterozygosity of microsatellites) is likely to be higher in the test subject than in the control subject. On the other hand, if the level of the LINE-1 DNA in the test sample is lower than in the control sample, the cancer is likely to be at a less advanced stage in the test subject than in the control subject, the test subject is likely to be more responsive to a cancer therapy than the control subject, the test subject is likely to have an increased probability of survival than the control subject, or the tumor genetic instability is likely to be lower in the test subject than in the control subject.

For example, if the level of the LINE-1 DNA in the test sample is higher than in the control sample, the level of RASSF1a, RARb, GSTP1, or MGMT gene unmethylated at the promoter region is likely to be higher in the test sample than in the control sample. Conversely, if the level of the LINE-1 DNA in the test sample is lower than in the control sample, the level of RASSF1a, RARb, GSTP1, or MGMT gene unmethylated at the promoter region is likely to be lower in the test sample than in the control sample.

In particular, in prostate cancer, if the level of the LINE-1 DNA in the test sample is higher than in a control sample from a control subject suffering from a multifocal prostate cancer, the test subject is likely to be suffering from a unifocal prostate cancer. If the level of the LINE-1 DNA in the test sample is lower than in a control sample from a control subject suffering from a unifocal prostate cancer, the test subject is likely to be suffering from a multifocal prostate cancer.

Moreover, if the level of the LINE-1 DNA in the test sample is higher than in the control sample, the prostate volume is likely to be larger in the test subject than in the control subject, or the PSA density is likely to be higher in the test subject than in the control subject. If the level of the LINE-1 DNA in the test sample is lower than in the control sample, the prostate volume is likely to be smaller in the test subject than in the control subject, or the PSA density is likely to be lower in the test subject than in the control subject.

The discovery that the level of LINE-1 DNA is increased in esophageal cancer, colorectal cancer, melanoma, and breast cancer cells is useful for identifying candidate compounds for treating cancer. Briefly, a esophageal cancer, colorectal cancer, melanoma, or breast cancer cell is contacted with a test compound. The level of LINE-1 DNA in the cell prior to and after the contacting step are compared. If the level of the LINE-1 DNA in the cell decreases after the contacting step, the test compound is identified as a candidate for treating cancer.

The test compounds can be obtained using any of the numerous approaches (e.g., combinatorial library methods) known in the art. See, e.g., U.S. Patent No. 6,462,187. Such libraries include, without limitation, peptide libraries, peptoid libraries (libraries of molecules having the functionalities of peptides, but with a novel, non-peptide backbone that is resistant to enzymatic degradation), spatially addressable parallel solid phase or solution phase libraries, synthetic libraries obtained by deconvolution or affinity chromatography selection, and the "one-bead one-compound" libraries. Compounds in the last three libraries can be peptides, non-peptide oligomers, or small molecules. Examples of methods for synthesizing molecular libraries can be found in the art. Libraries of compounds may be presented in solution, or on beads, chips, bacteria, spores, plasmids, or phages.

The compounds so identified are within the invention. These compounds and other compounds known to promote DNA methylation or inhibit demethylation of DNA can be used for treating cancer by administering an effective amount of such a compound to a subject suffering from cancer (e.g., prostate cancer, esophageal cancer, colorectal cancer, melanoma, or breast cancer).

A subject to be treated may be identified in the judgment of the subject or a health care professional, and can be subjective (e.g., opinion) or objective (e.g., measurable by a test or diagnostic method such as those described above).

A "treatment" is defined as administration of a substance to a subject with the purpose to cure, alleviate, relieve, remedy, prevent, or ameliorate a disorder, symptoms of the disorder, a disease state secondary to the disorder, or predisposition toward the disorder.

An "effective amount" is an amount of a compound that is capable of producing a medically desirable result in a treated subject. The medically desirable result may be objective (i.e., measurable by some test or marker) or subjective (i.e., subject gives an indication of or feels an effect).

For treatment of cancer, a compound is preferably delivered directly to tumor cells, e.g., to a tumor or a tumor bed following surgical excision of the tumor, in order to treat any remaining tumor cells. For prevention of cancer invasion and metastases, the compound can be administered to, for example, a subject that has not yet developed detectable invasion and metastases but is found to have an increased level of LINE-1 DNA.

The identified compounds can be incorporated into pharmaceutical compositions. Such compositions typically include the compounds and pharmaceutically acceptable carriers. "Pharmaceutically acceptable carriers" include solvents, dispersion media, coatings, antibacterial and antifungal agents, isotonic and absorption delaying agents, and the like, compatible with pharmaceutical administration.

A pharmaceutical composition is formulated to be compatible with its intended route of administration. See, e.g., U.S. Patent No. 6,756,196. Examples of routes of administration include parenteral, e.g., intravenous, intradermal, subcutaneous, oral (e.g., inhalation), transdermal (topical), transmucosal, and rectal administration.

It is advantageous to formulate oral or parenteral compositions in dosage unit form for ease of administration and uniformity of dosage. "Dosage unit form," as used herein, refers to physically discrete units suited as unitary dosages for the subject to be treated, each unit containing a predetermined quantity of an active compound calculated to produce the desired therapeutic effect in association with the required pharmaceutical carrier.

The dosage required for treating a subject depends on the choice of the route of administration, the nature of the formulation, the nature of the subject's illness, the subject's size, weight, surface area, age, and sex, other drugs being administered, and the judgment of the attending physician. Suitable dosages are in the range of 0.01-100.0 mg/kg. Wide variations in the needed dosage are to be expected in view of the variety of compounds available and the different efficiencies of various routes of administration. For example, oral administration would be expected to require higher dosages than administration by intravenous injection. Variations in these dosage levels can be adjusted using standard empirical routines for optimization as is well understood in the art. Encapsulation of the compound in a suitable delivery vehicle (e.g., polymeric microparticles or implantable devices) may increase the efficiency of delivery, particularly for oral delivery.

The following examples are intended to illustrate, but not to limit, the scope of the invention. While such examples are typical of those that might be used, other procedures known to those skilled in the art may alternatively be utilized. Indeed, those of ordinary skill in the art can readily envision and produce further embodiments, based on the teachings herein, without undue experimentation.

### EXAMPLES

### A. PROSTATE CANCER STUDIES

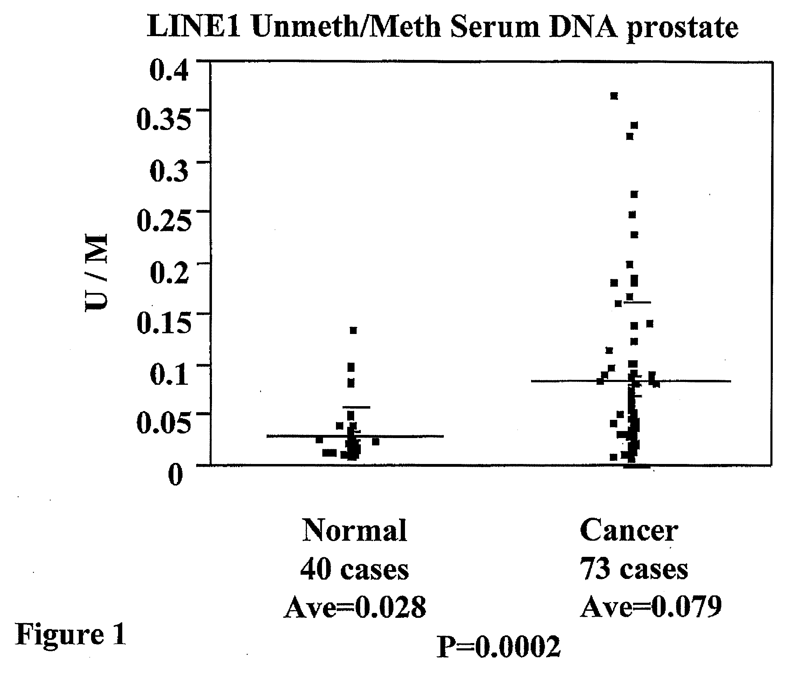

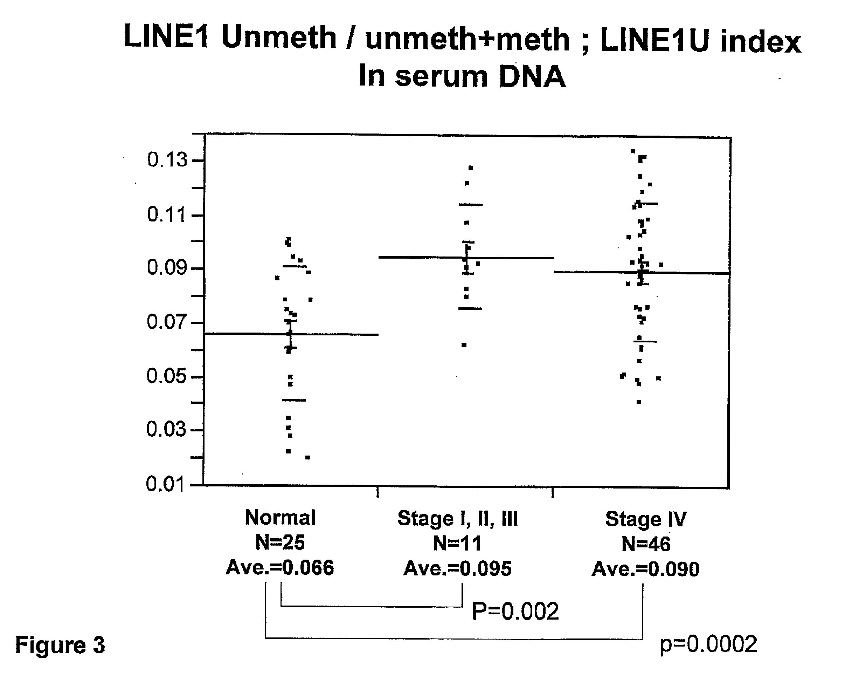

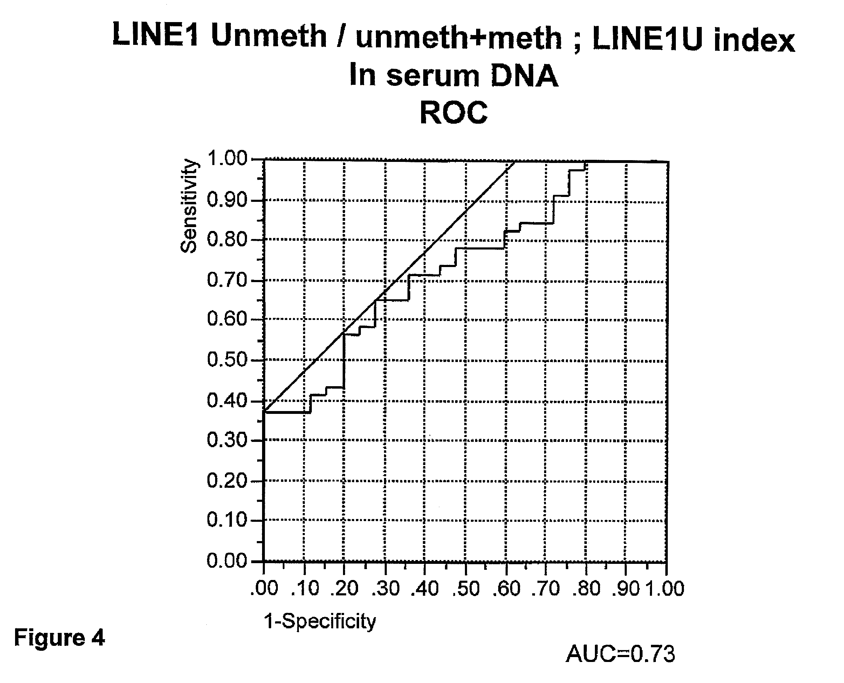

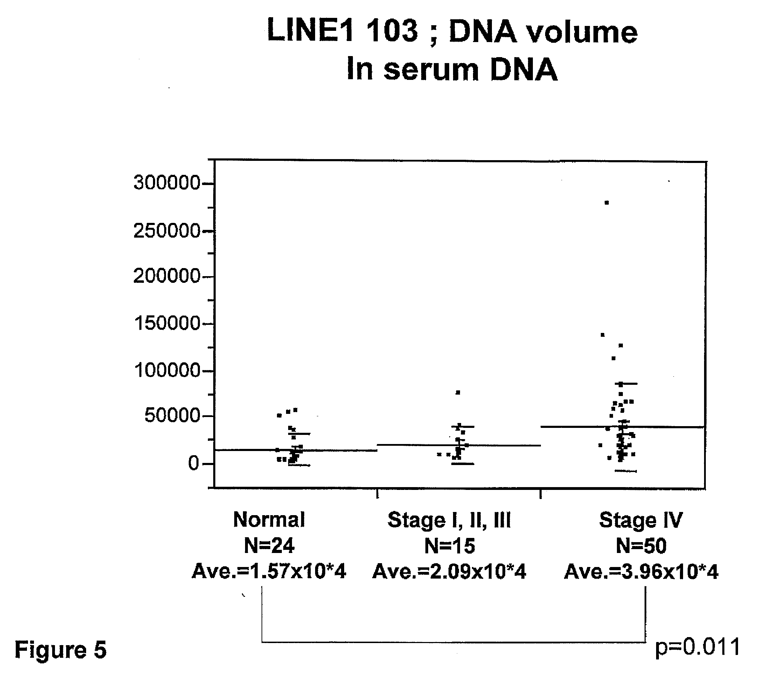

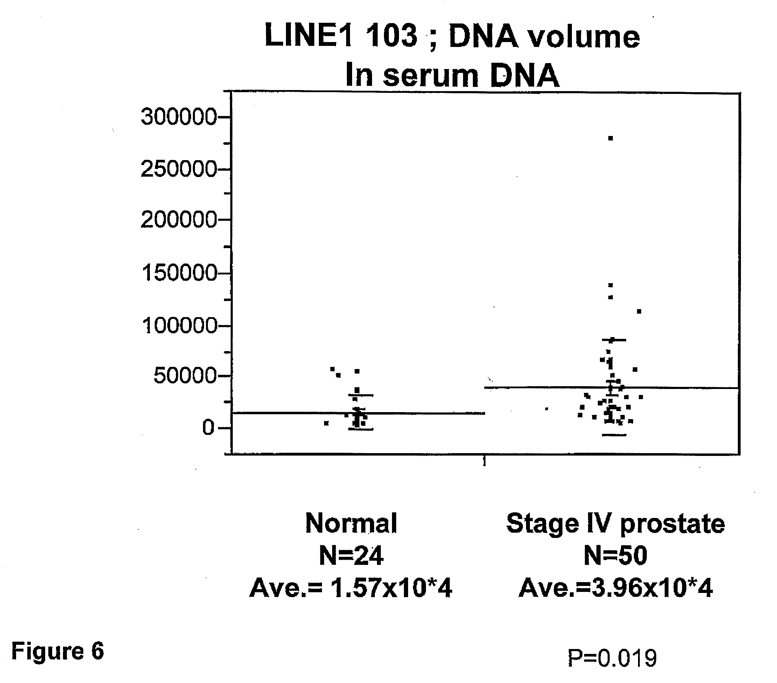

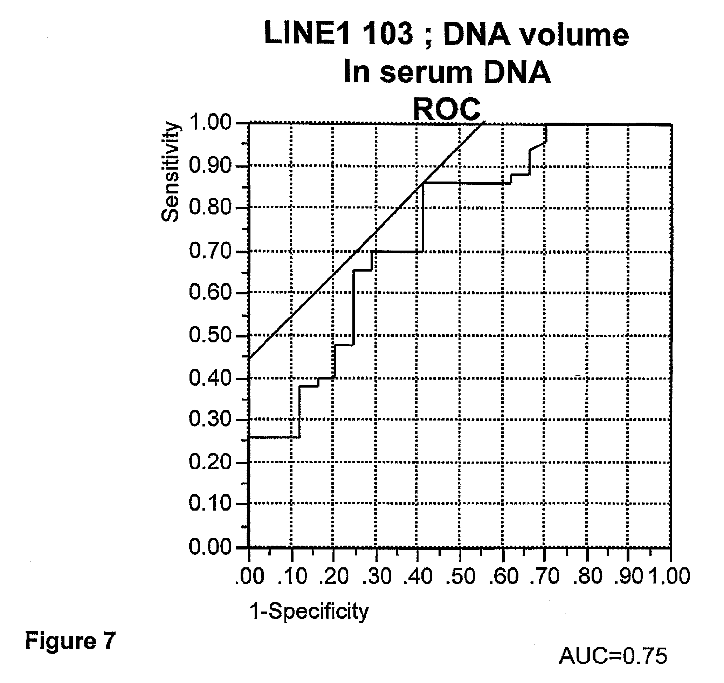

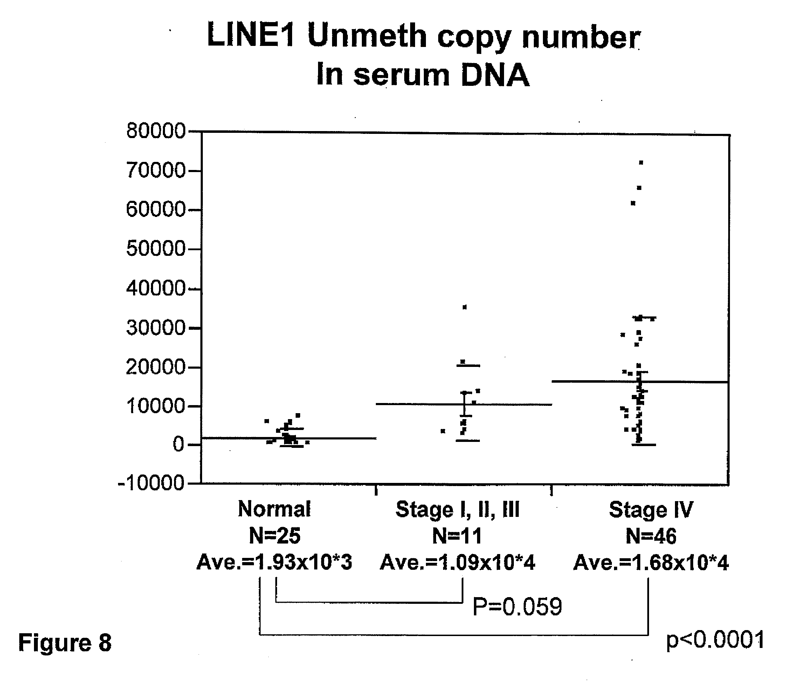

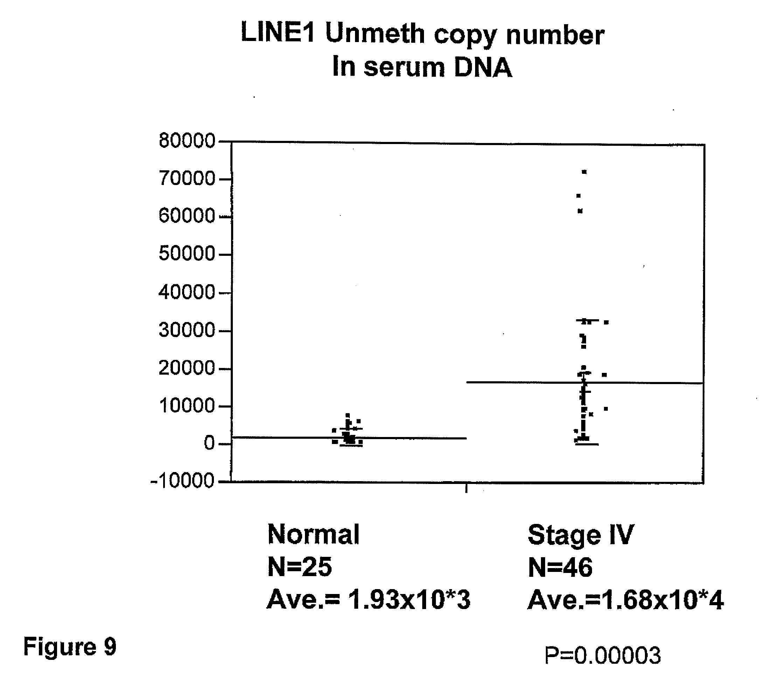

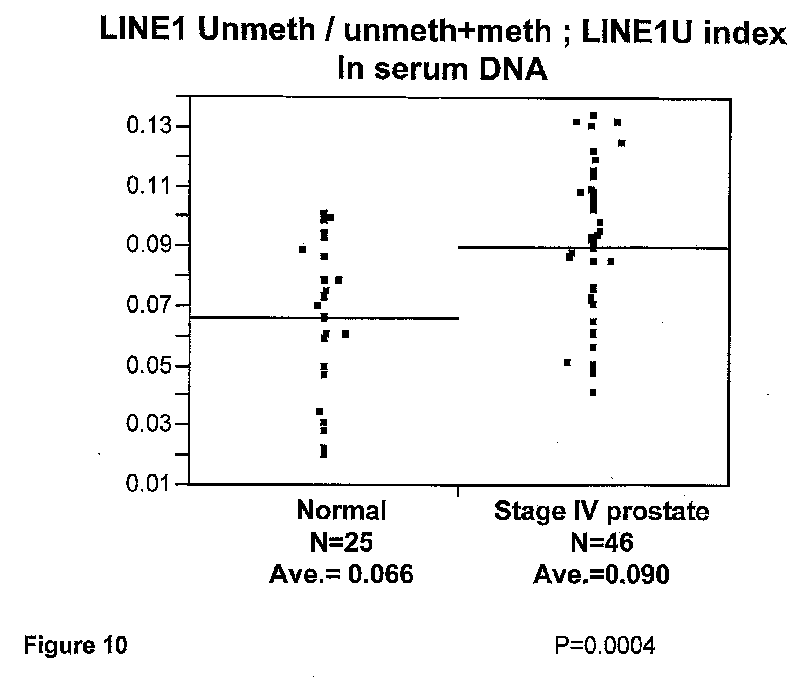

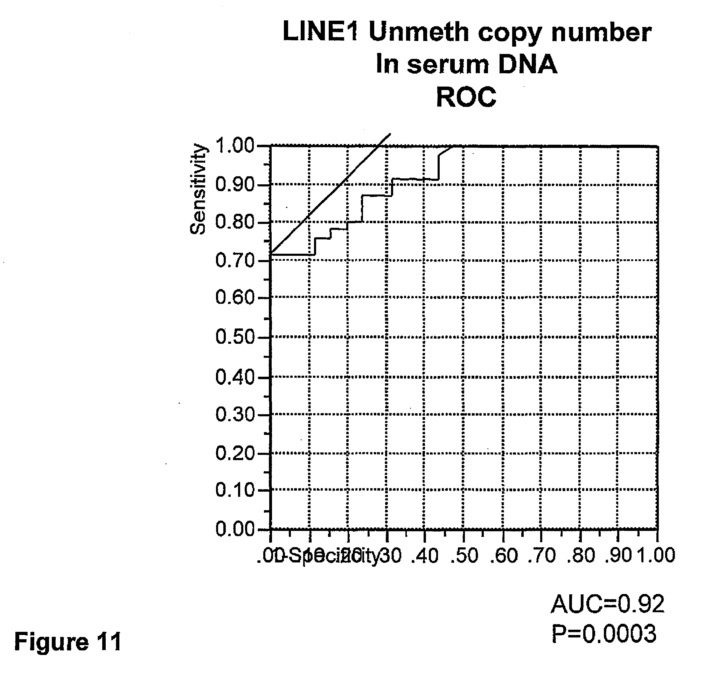

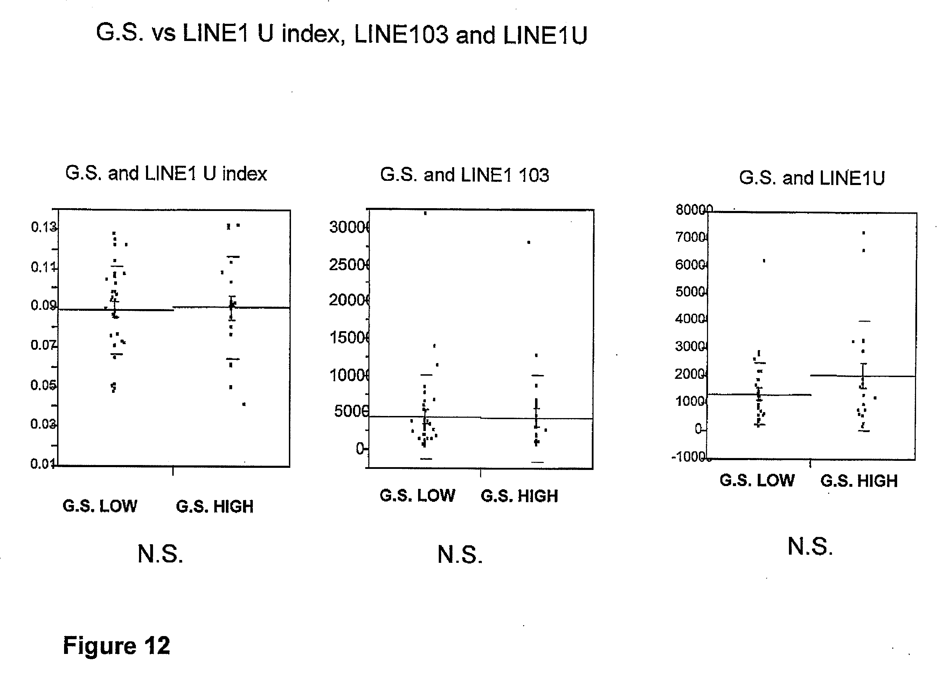

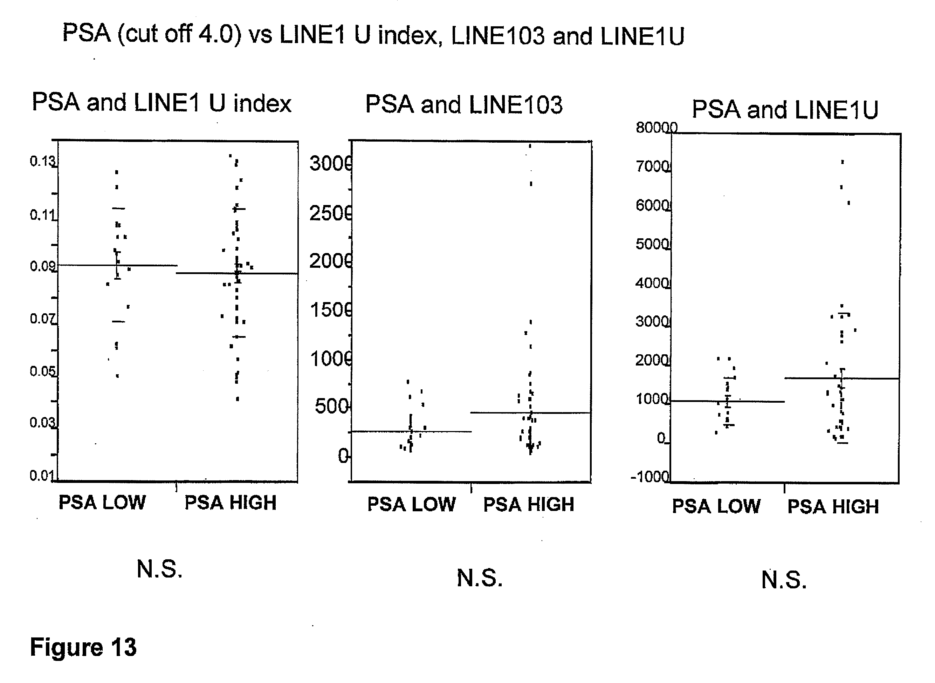

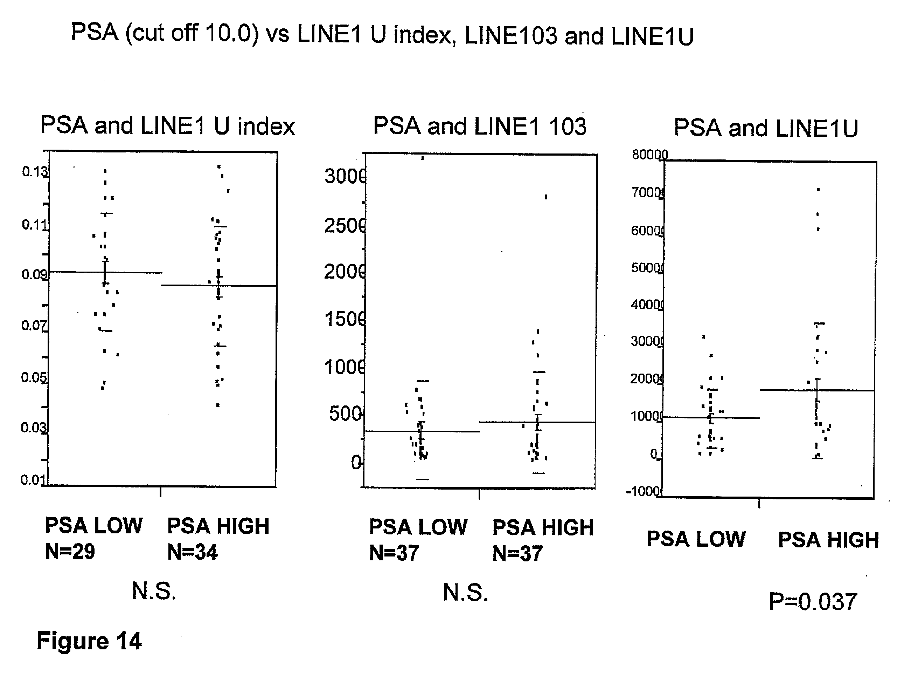

### 1. LINE1 DNA prepared from body fluids (Figures 1-14)

DNA extraction from serum/plasma. Blood was drawn for serum before operation or starting any treatment. Ten milliliters of blood were collected in serum separator tubes, centrifuged, filtered through a 13 µm-serum filter, aliquoted, and cryopreserved at -30 °C. DNA was extracted from 500 µL of serum using SDS and Proteinase K.

Sodium bisulfite modification (SBM of serum/plasma DNA. Extracted DNA was subjected to sodium bisulfite modification. DNA was denatured in 0.3 mol/L NaOH for 3 minutes at 95°C. Sodium bisulfite modification was performed at 60 °C for 3 hours by adding 550 µl of 2.5 mol/L sodium bisulfite and 125 nmol/L hydroquinone solution. Salts were removed using the Wizard DNA Clean-up System and desulfonated in 0.3 mol/ L NaOH at 37°C for 15 minutes.

Quantitative real-time PCR using real-time PCR or AQAMA for LINE1 promoter region analysis. The copy number of both methylated and unmethylated LINE1 genes were calculated by fluorescence-based real-time quantitative methylation specific PCR. Specific amplification primer sets and amplicon-specific fluorogenic hybridization probes were designed for both bisulfite-converted methylated and unmethylated sequence of LINE1 promoter region. As a control, specific plasmids for both methylated and unmethylated LINE1 were prepared. Separate fluorogenic quantitative real-time MSP were performed for both methylated and unmethylated LINE1 promoter regions using ABI 7900 Thermocycler or Icycler (BioRad). After quantifying the copy numbers of both methylated and unmethylated LINE1, the "unmethylation index" (copy number of unmethylation divided by total copy number) were calculated.

Analysis. Serum DNA from seventy-three prostate cancer patients and forty normal human males were collected. LINE1 unmethylation index between prostate cancer patients and normal human were compared. Among prostate cancer patients, the relationship between LINE1 methylation status and other clinico-pathological data was analyzed. The results are presented in Figures 1-2 and described under "BRIEF

### DESCRIPTION OF THE FIGURES."

As shown in Figures 1-2, serum DNA from prostate cancer patients showed significantly higher LINE1 unmethylation index than those from normal human. LINE1 unmethylation index of serum DNA from prostate cancer patients correlates with the methylation status of other cancer-related genes.

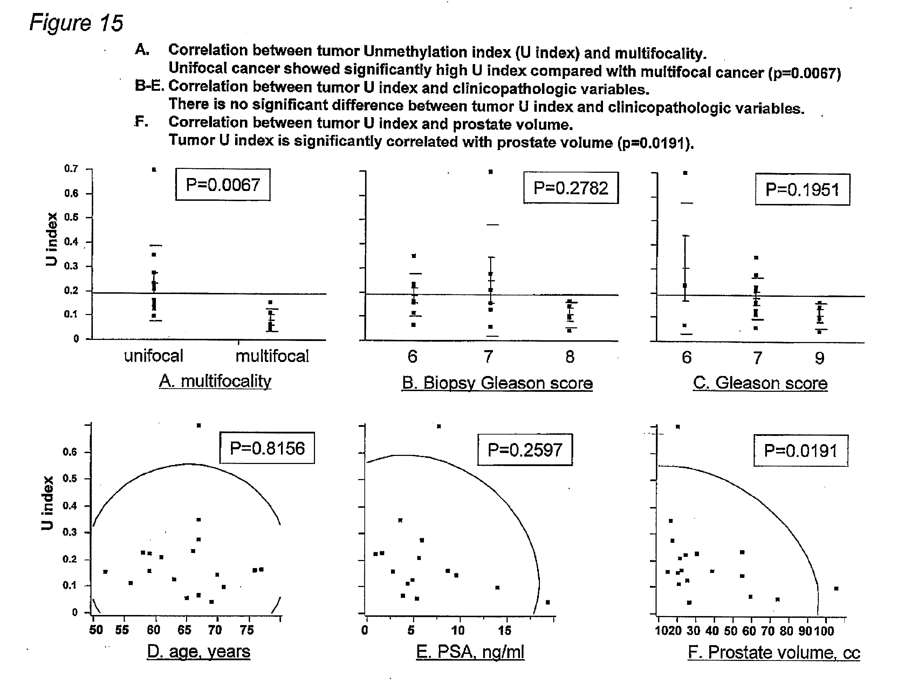

### 2. LINE1 DNA prepared from tissue samples (Figure 15)

| | |
|---|---|
| pilot study population: | 18 prostate cancer paraffin tissues (matched adjacent normal tissues) |
| DNA extraction: | microdissection & PCI |
| U index quantification: | AQAMA |
| | U index = unmeth / meth + unmeth |
| Statistical analysis: | unpaired t test |
| | Spearman rank correlation |

As shown in Figure 15, tumors tend to show higher U index compared with normal tissue. However, there is no significant difference. Since this pilot study number is small (n=18), further study should be required. On the other hand, unifocal cancer showed significantly high U index compared with multifocal cancer (p=0.0067). Tumor U index was also significantly correlated with prostate volume (p=0.0191), suggesting correlations between LINE1 U index and prostate volume-related markers (such as PSA density).

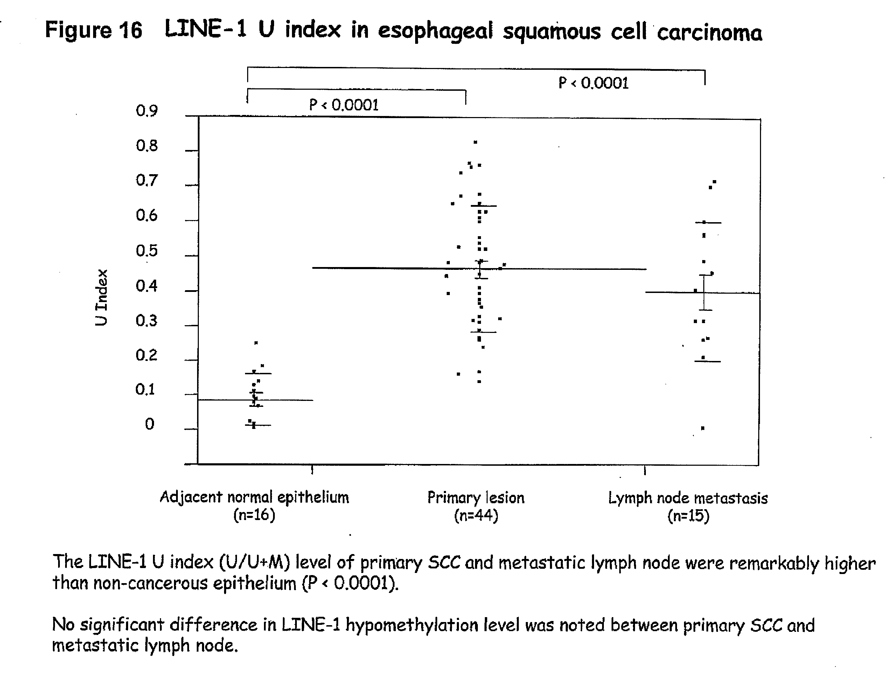

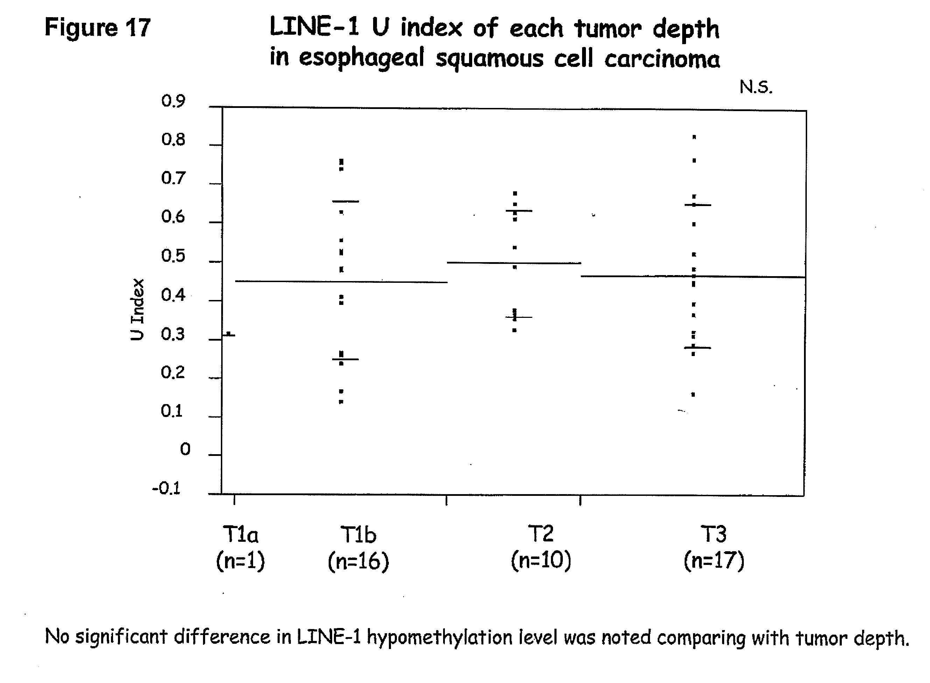

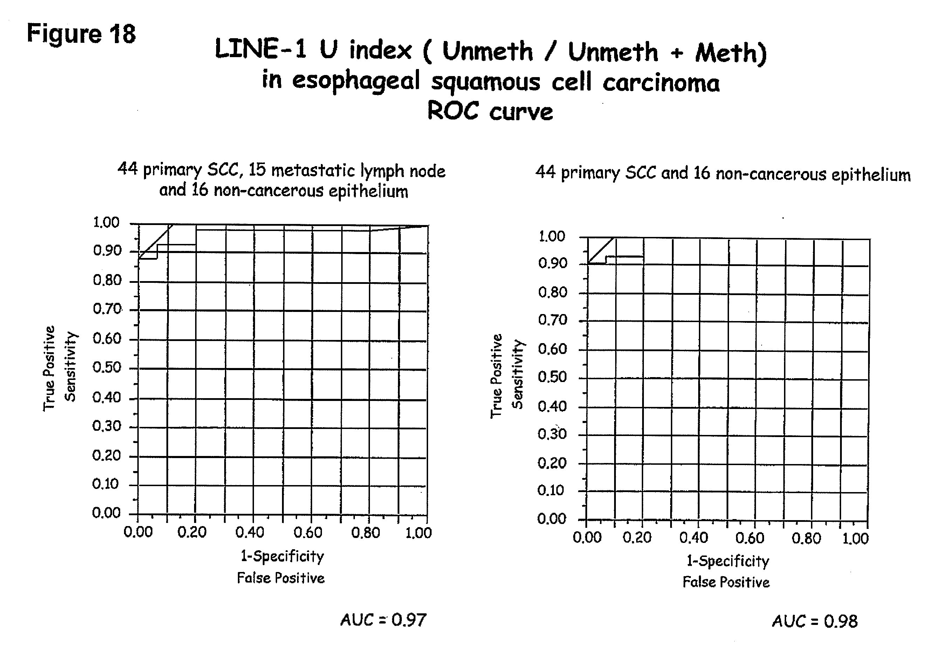

### B. HYPOMETHYLATION OF LINE-1 IN ESOPHAGEAL SQUAMOUS CELL CARCINOMA (FIGURES 16-18)

Objective. To evaluate characteristics of global hypomethylation in evolution of esophageal squamous cell carcinoma (SCC).

Materials and methods. 44 cases of SCC, 16 cases of non-cancerous epithelium, and 15 cases of metastatic lymph node were studied. Microdissection was performed to separate SCC, adjacent non-cancerous epithelium, and metastatic lymph node prior to DNA extraction. Hypomethylation levels of LINE-1 repetitive elements were measured by using absolute quantitative analysis of methylated alleles (AQAMA). The ratios of LINE-1 hypomethylation for SCC, non-cancerous epithelium, and lymph node metastasis were compared.

Results. The LINE-1 U index (U/U+W level of primary SCC and metastatic lymph node were remarkably higher than non-cancerous epithelium (P < 0.0001). No significant difference in LINE-1 hypomethylation level was noted between primary SCC and metastatic lymph node. No significant difference in LINE-1 hypomethylation level was noted comparing with tumor depth.

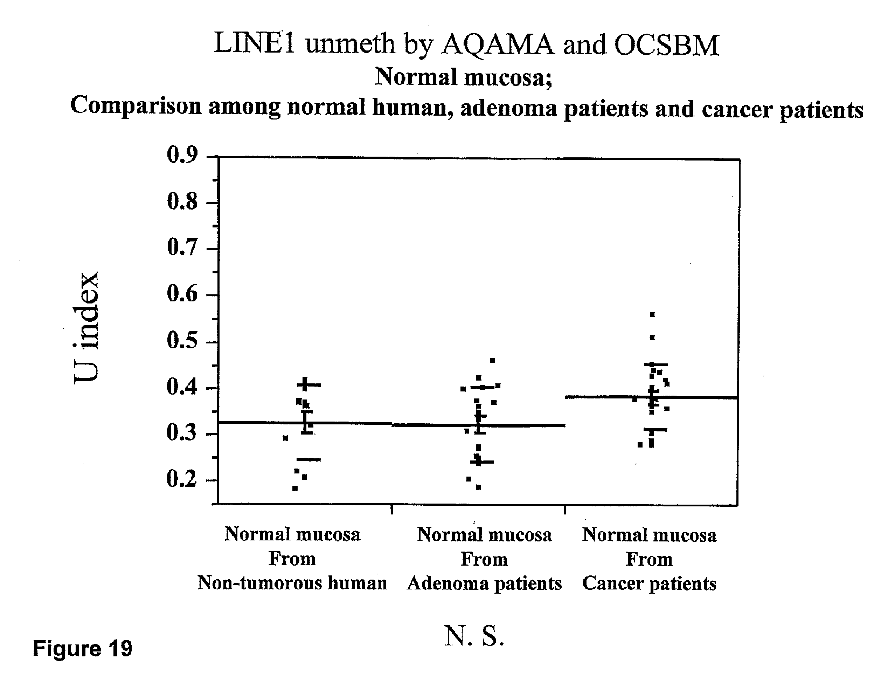

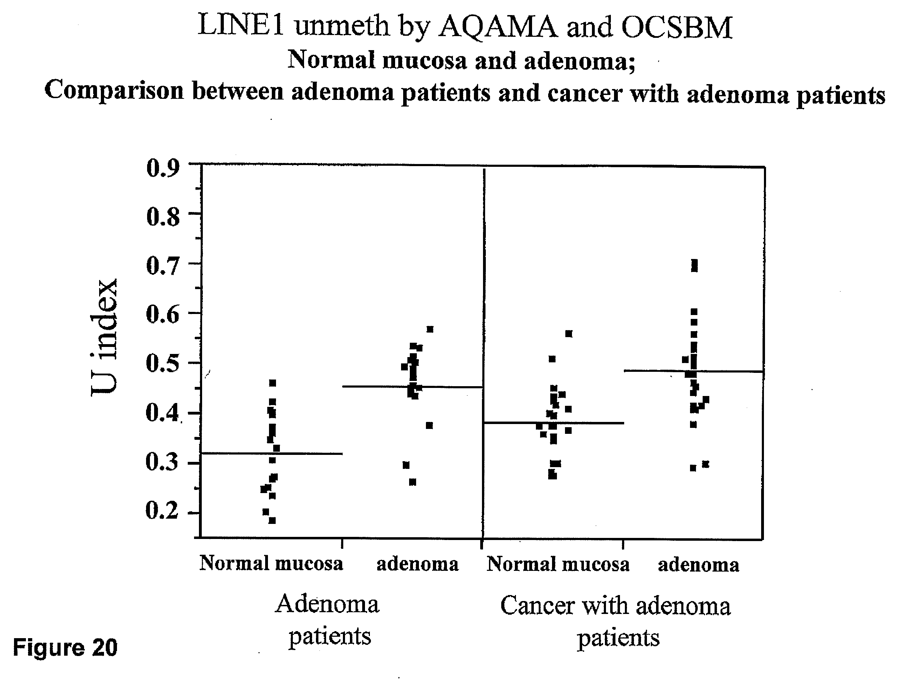

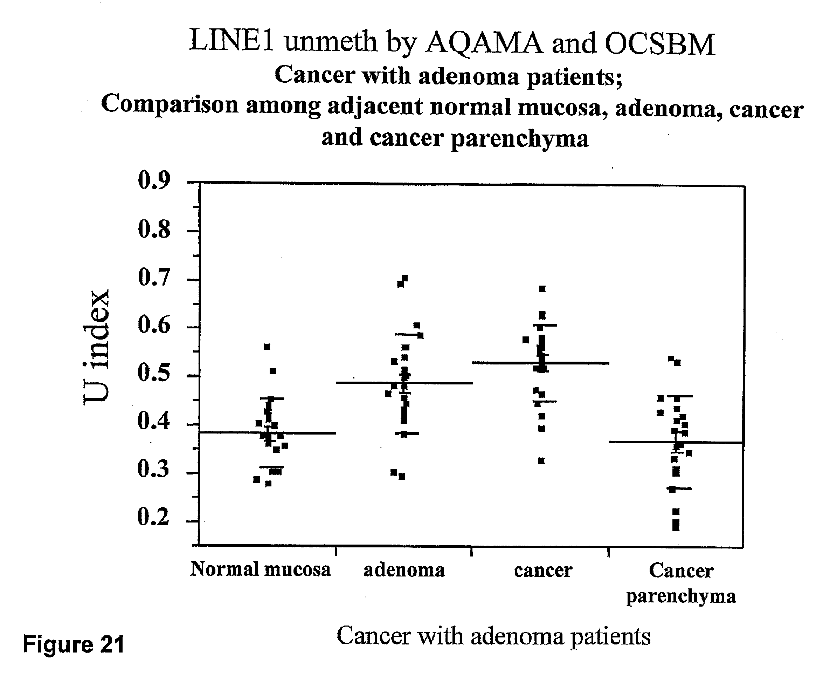

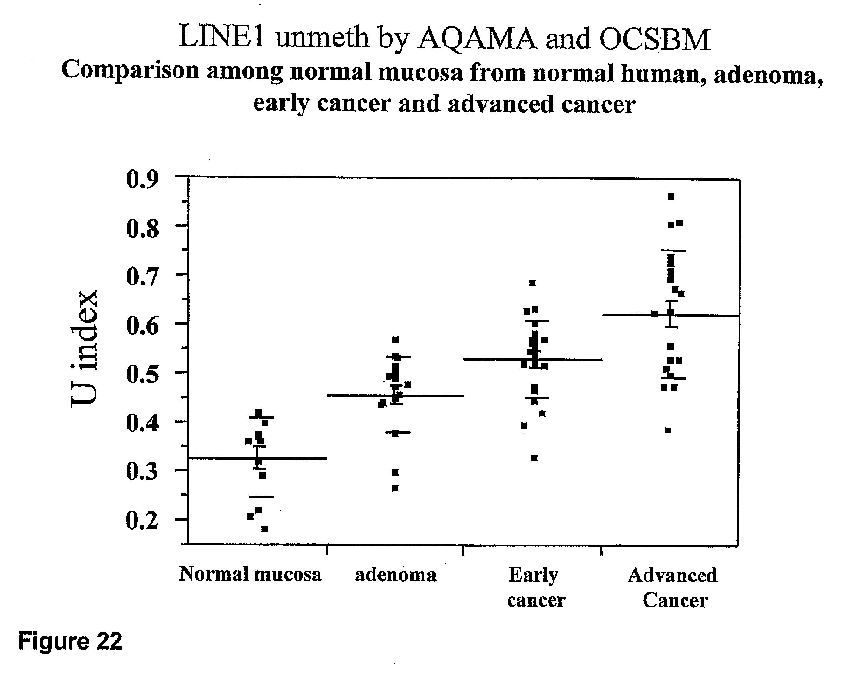

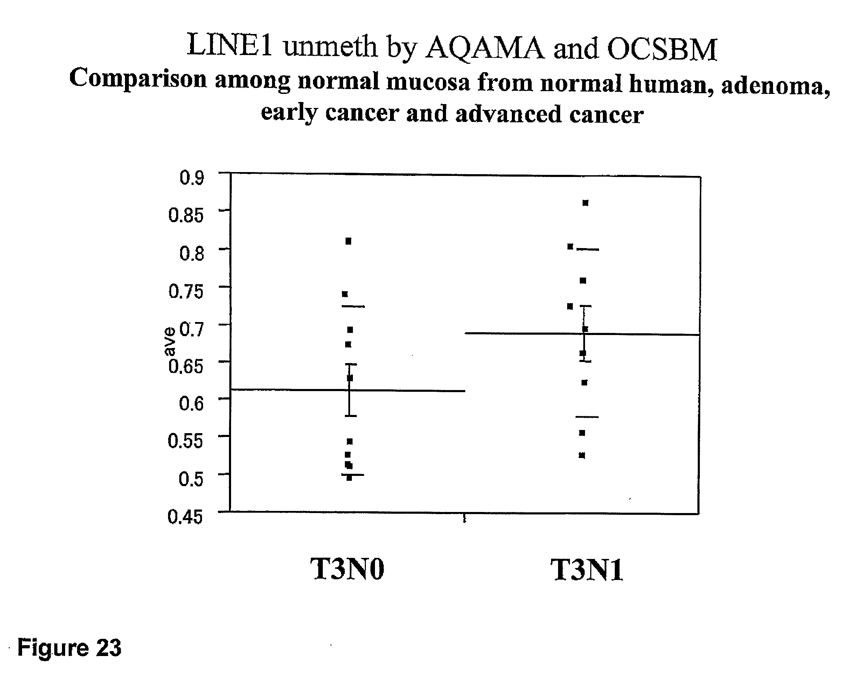

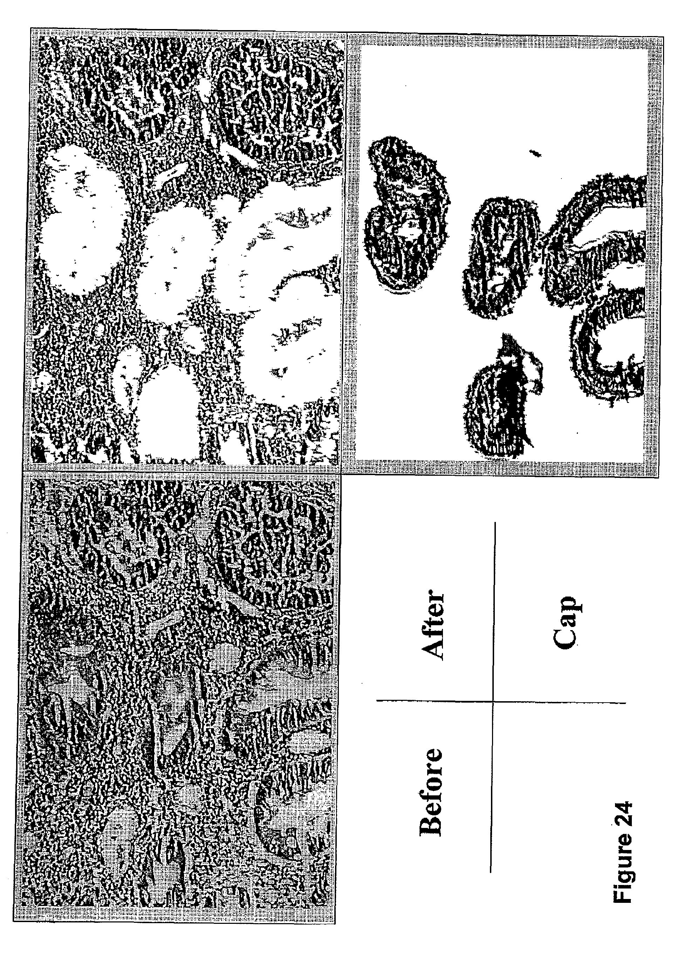

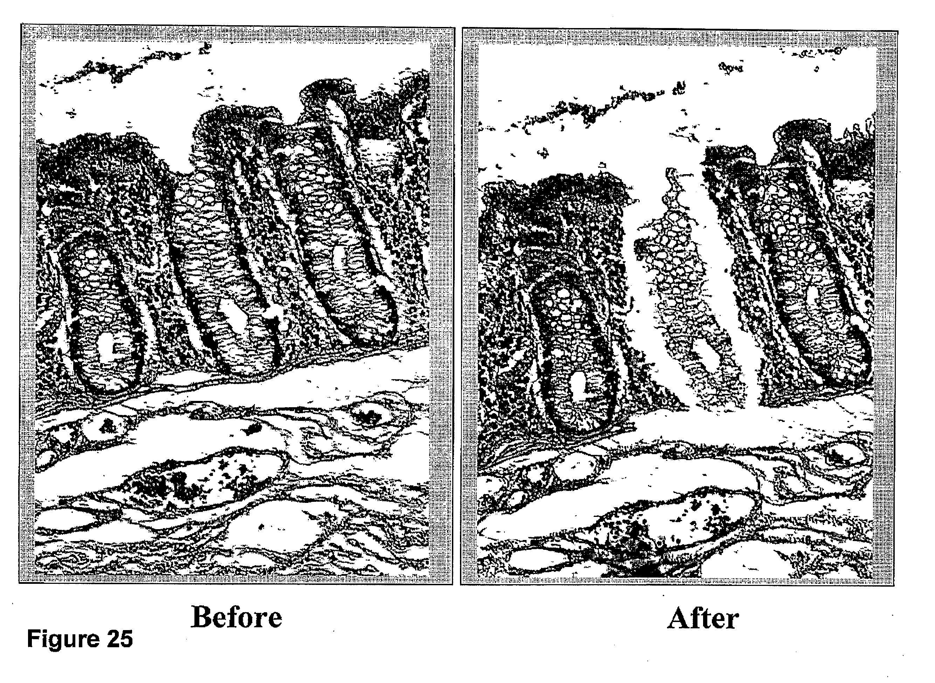

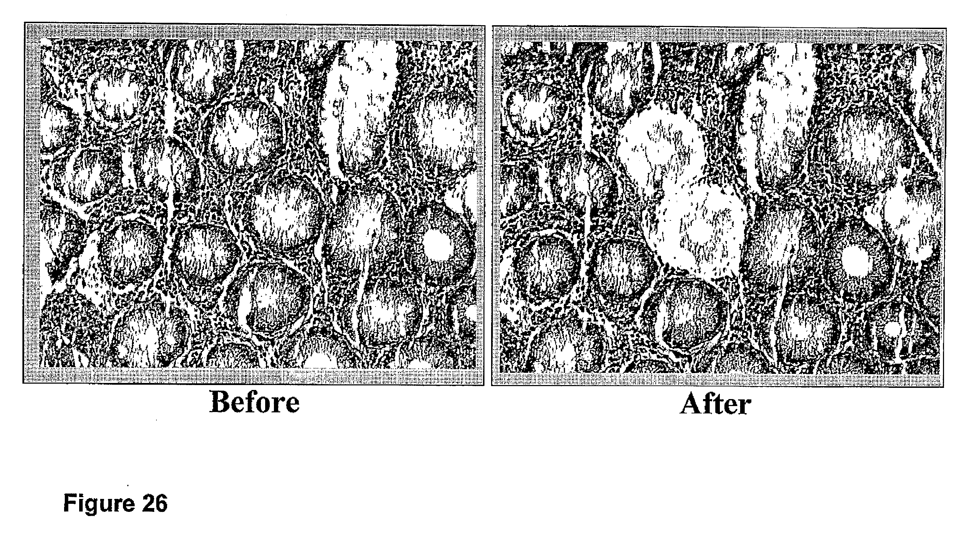

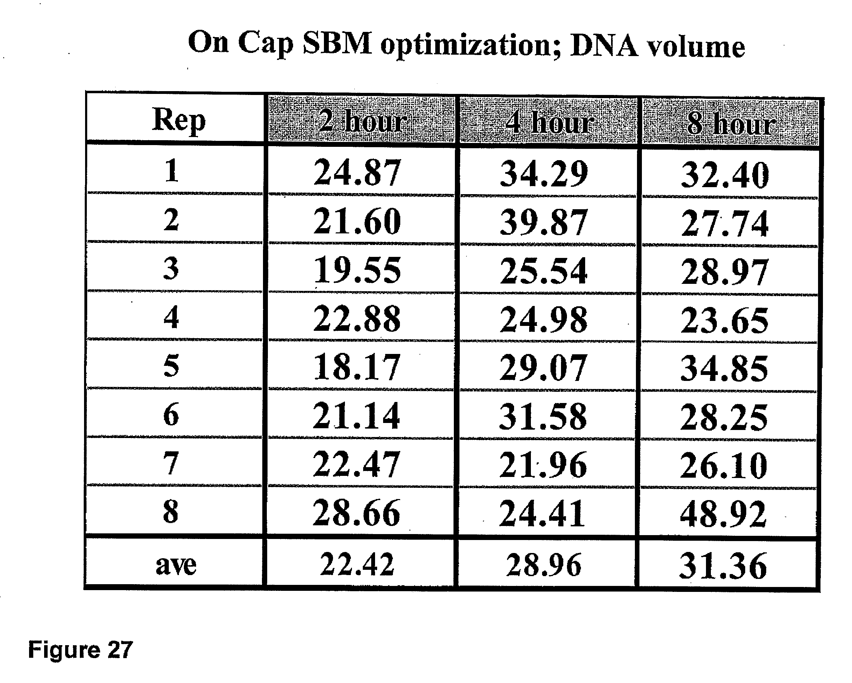

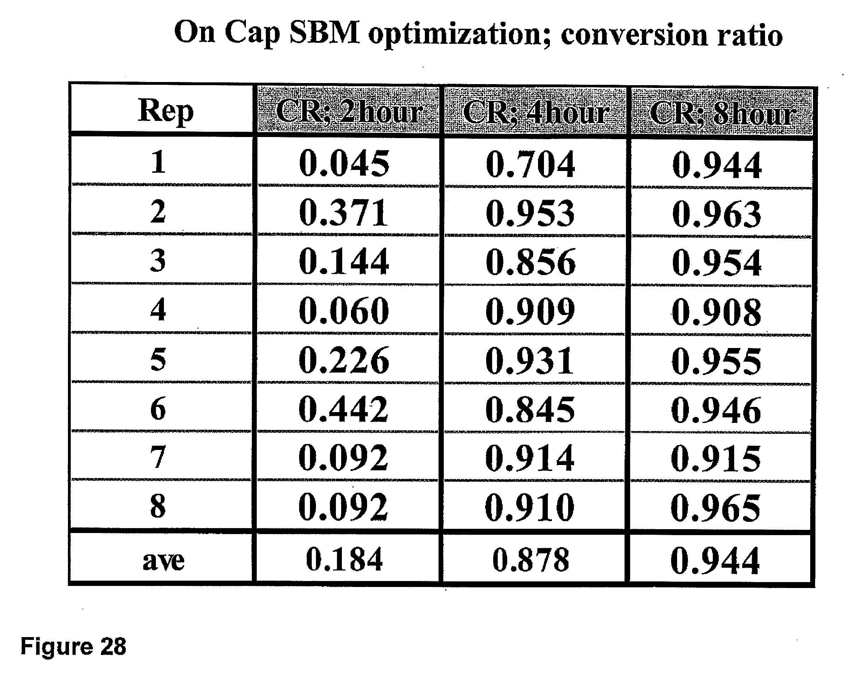

### C. HYPOMETHYLATION OF LINE-1 IN COLORECTAL CANCER (FIGURES 19-28)

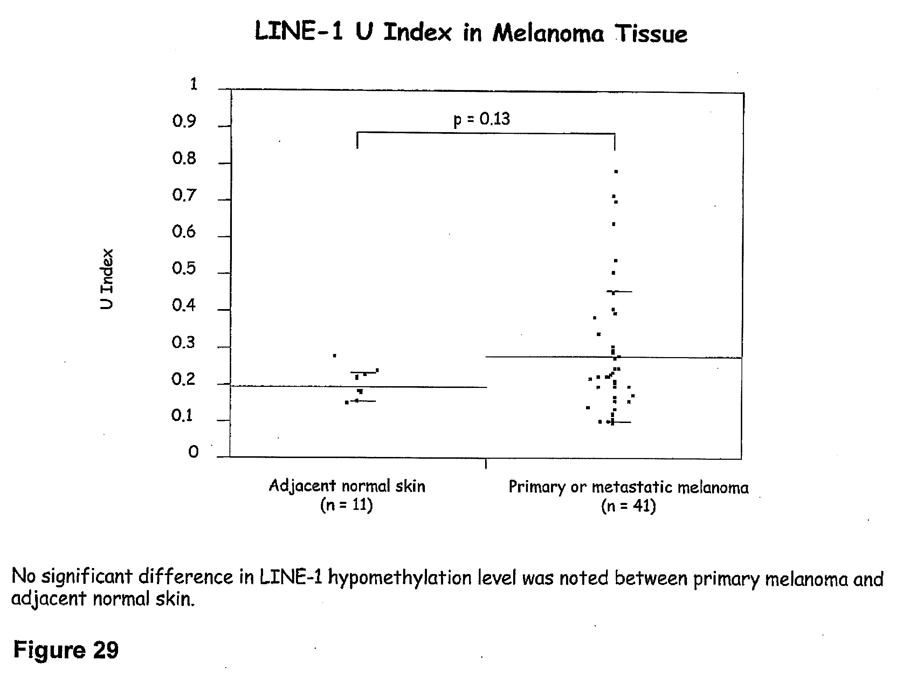

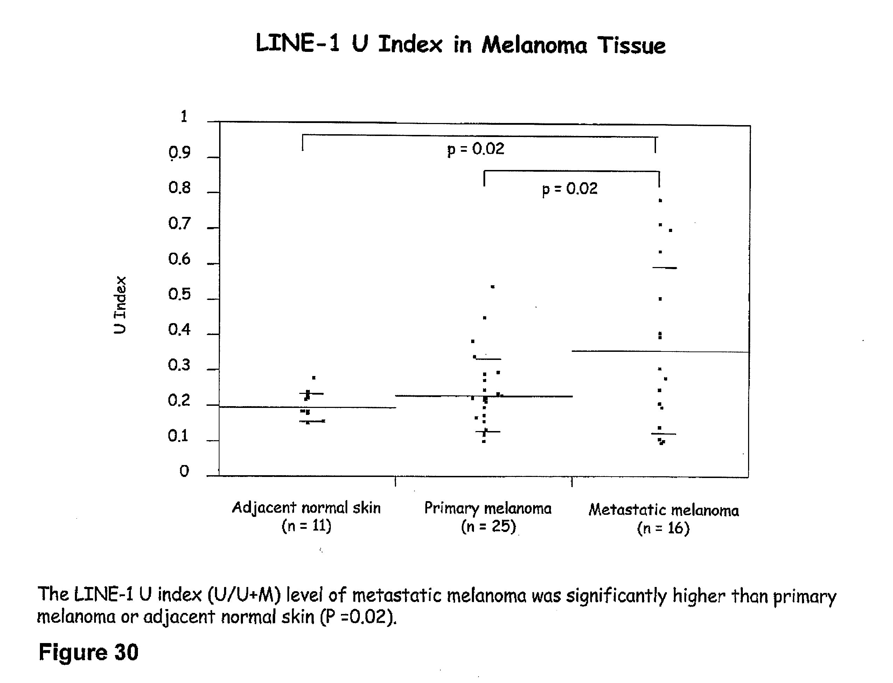

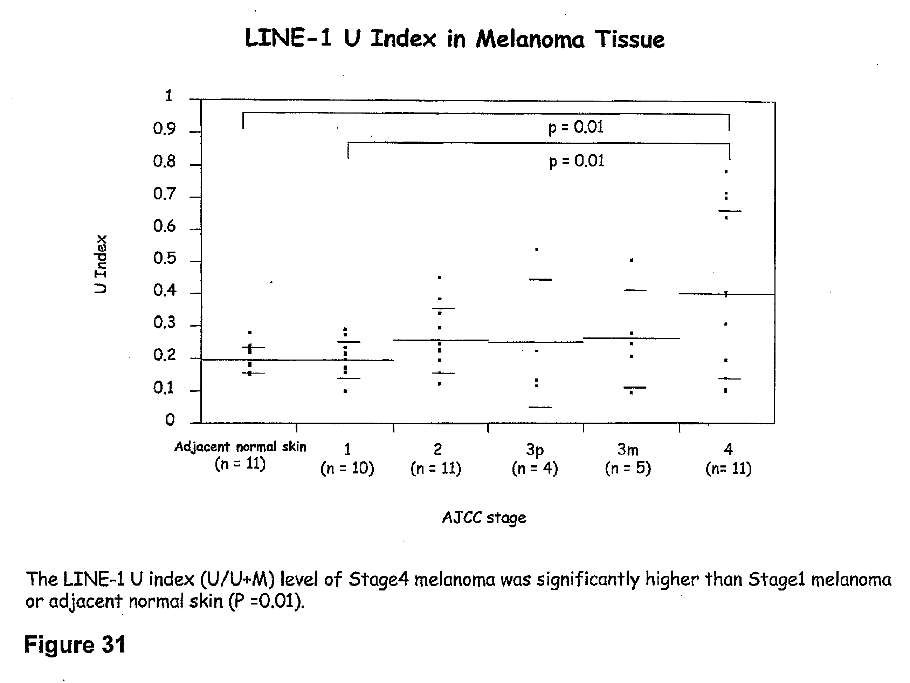

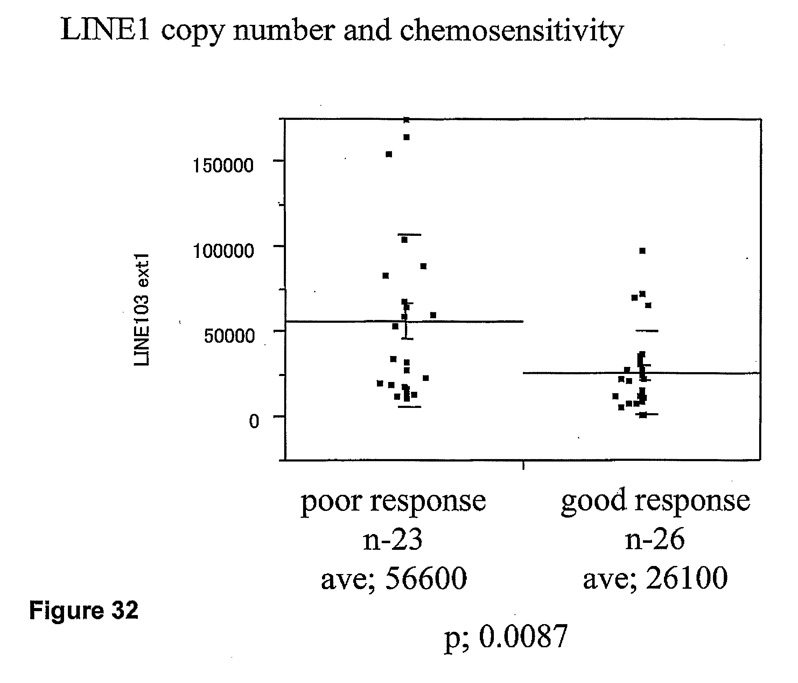

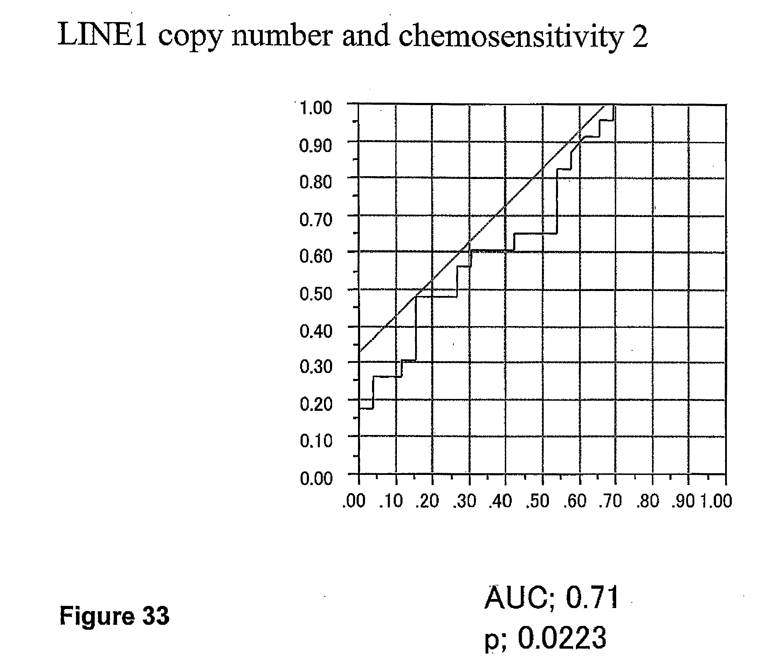

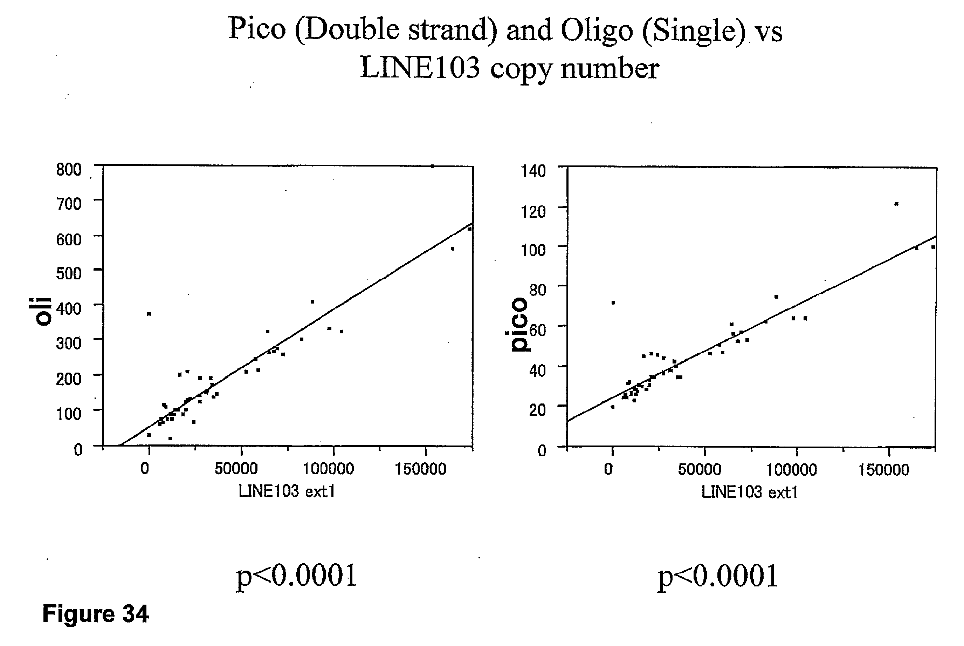

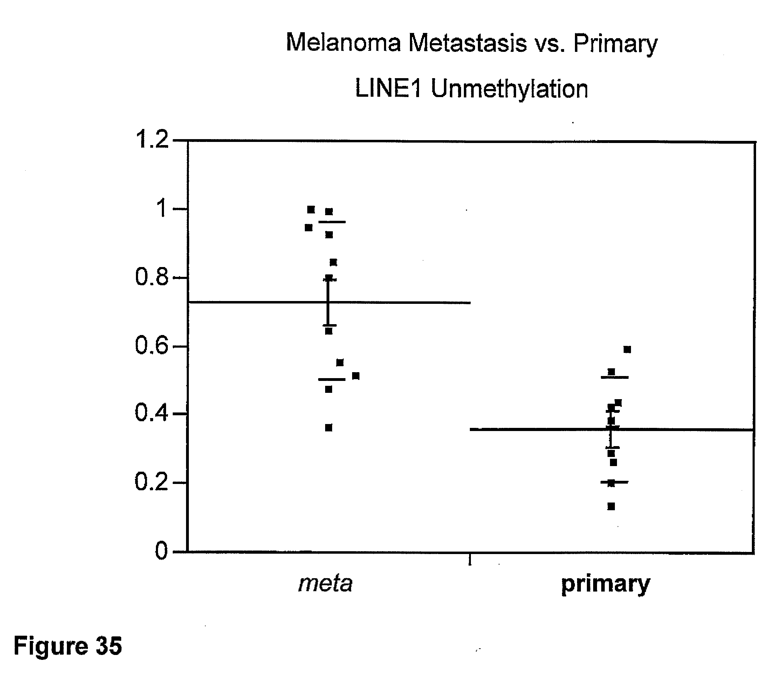

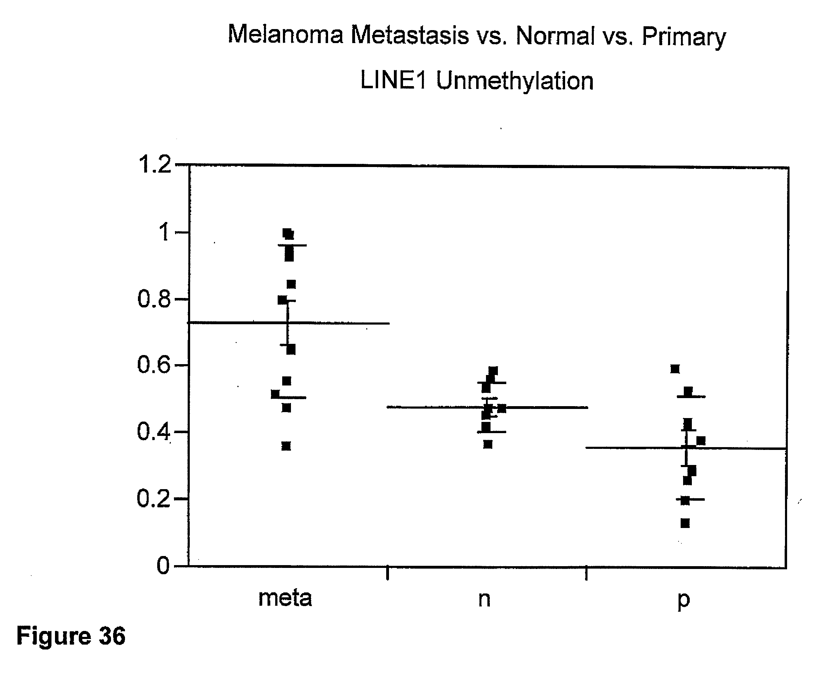

### D. HYPOMETHYLATION OF LINE-1 IN MELANOMA (FIGURES 29-36)

Objective. To evaluate characteristics of global hypomethylation in the development of melanoma.

Materials and methods. 41 cases of melanoma patients, 11 cases of adjacent normal skin, 25 cases of primary melanoma, and 16 cases of metastatic melanoma were studied. Microdissection was performed to separate melanoma, adjacent normal skin, and metastatic lymph node prior to DNA extraction. Hypomethylation levels of LINE-1 repetitive elements were measured by using absolute quantitative analysis of methylated alleles (AQAMA). The ratios of LINE-1 hypomethylation for primary melanoma, adjacent normal skin, and metastatic lesions were compared.

Results. The LINE-1 U index (U/U+M) level of metastatic melanoma was significantly higher than primary melanoma or adjacent normal skin (P =0.02). No significant difference in LINE-1 hypomethylation level was noted between primary melanoma and adjacent normal skin. The LINE-1 U index (U/U+M) level of Stage4 melanoma was significantly higher than Stage1 melanoma or adjacent normal skin (P =0.01).

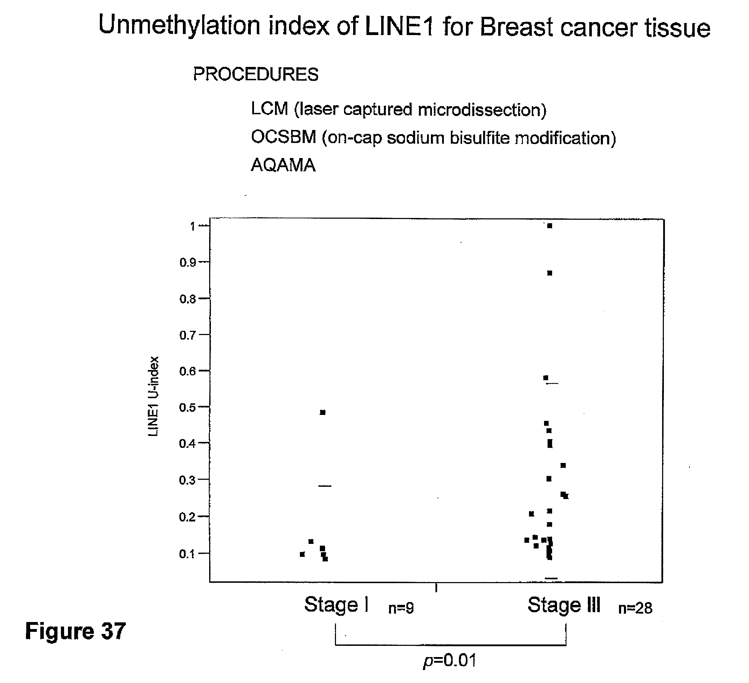

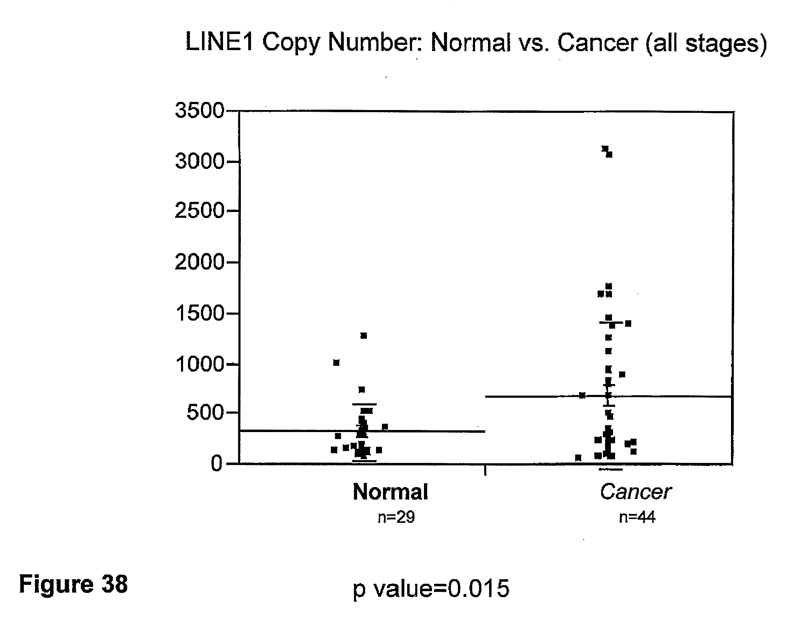

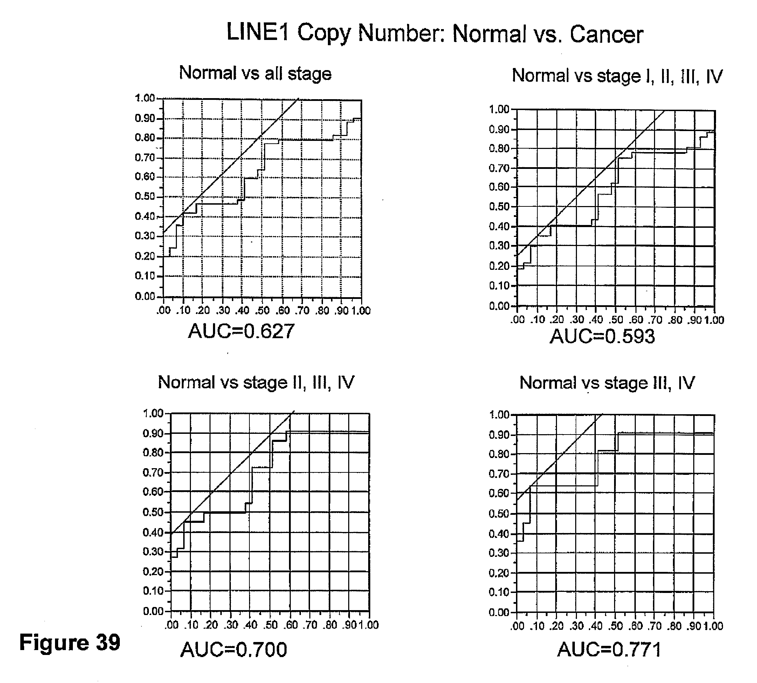

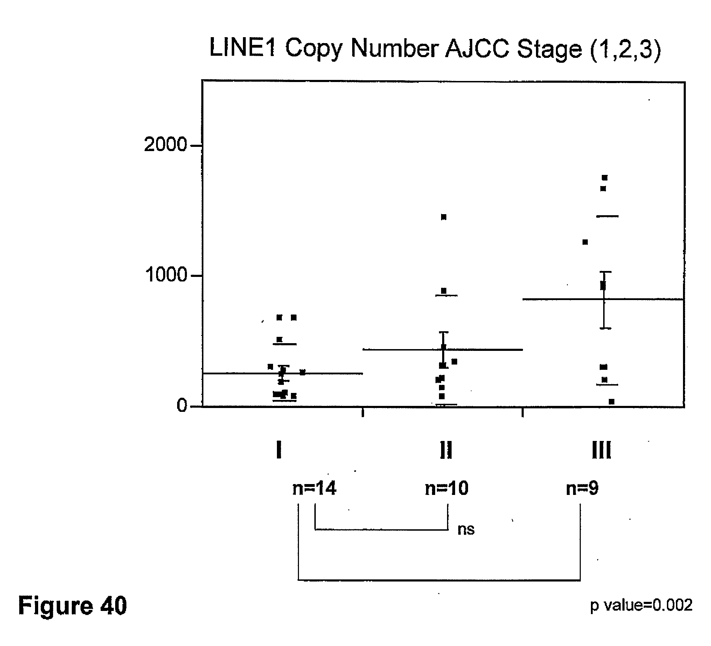

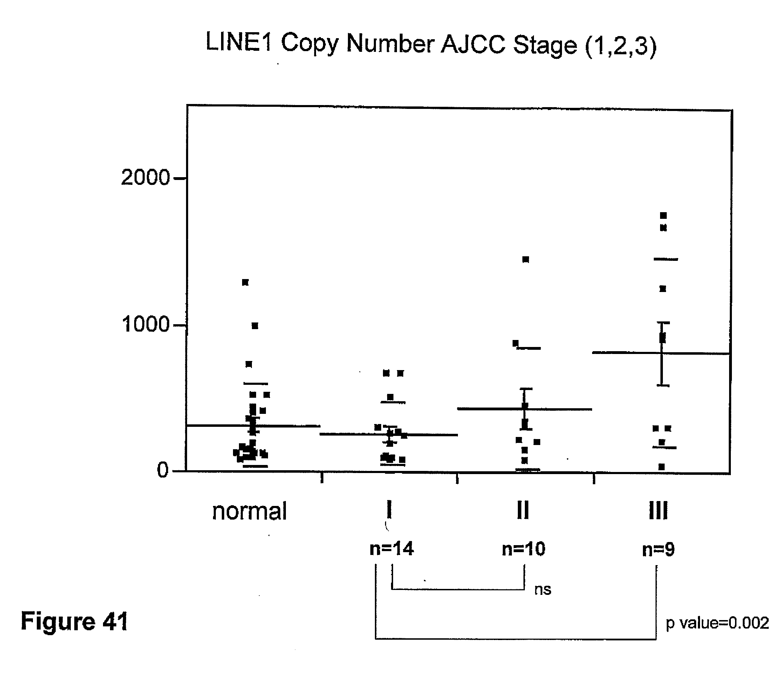

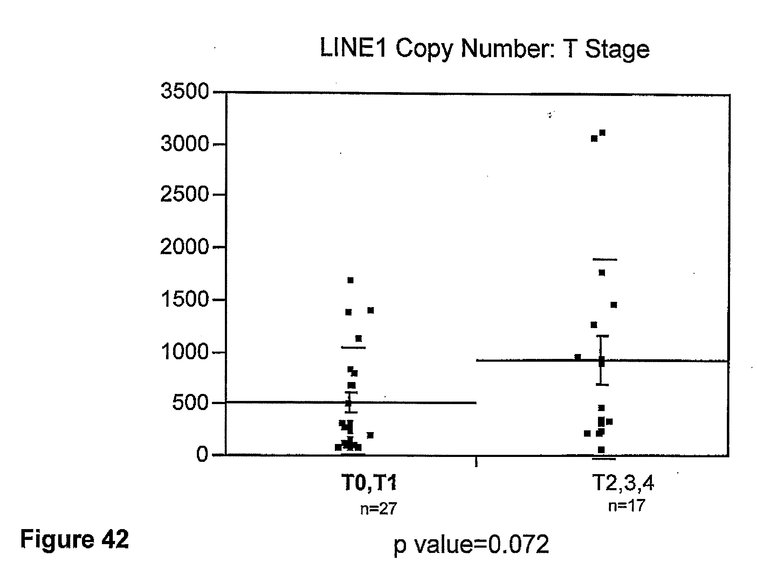

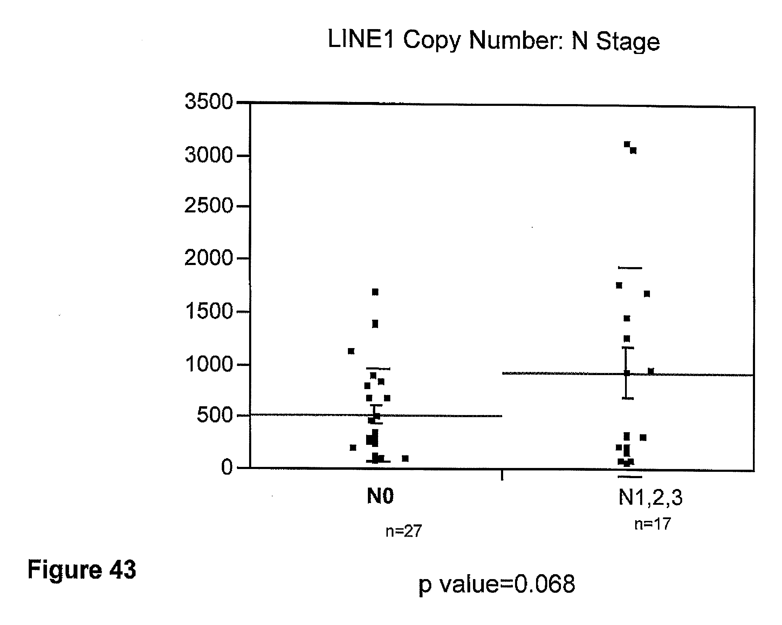

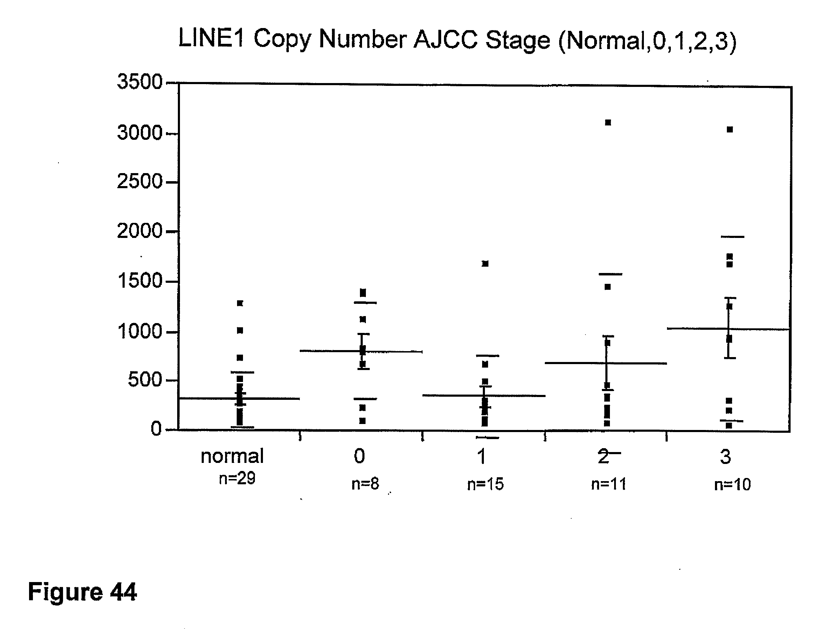

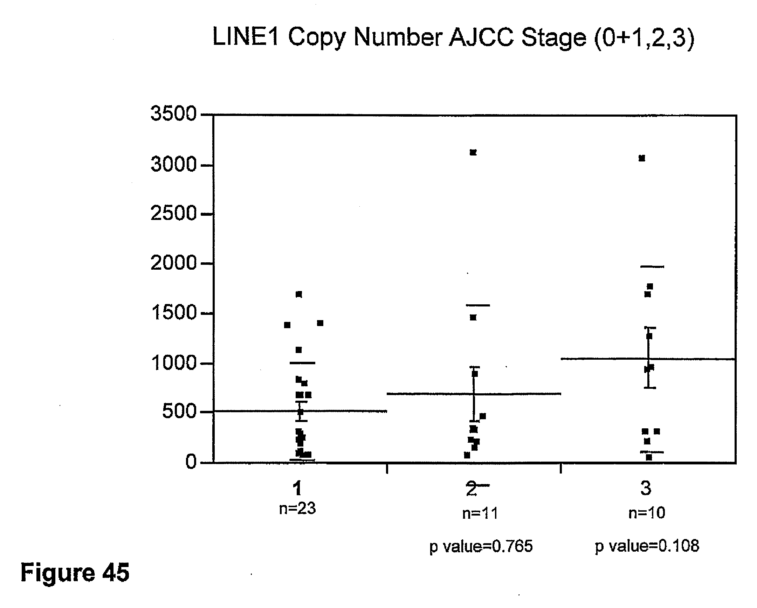

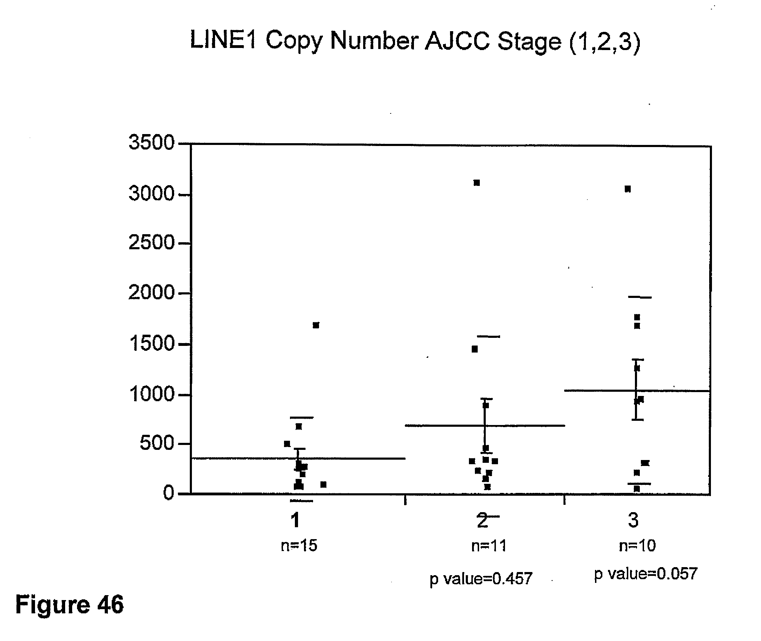

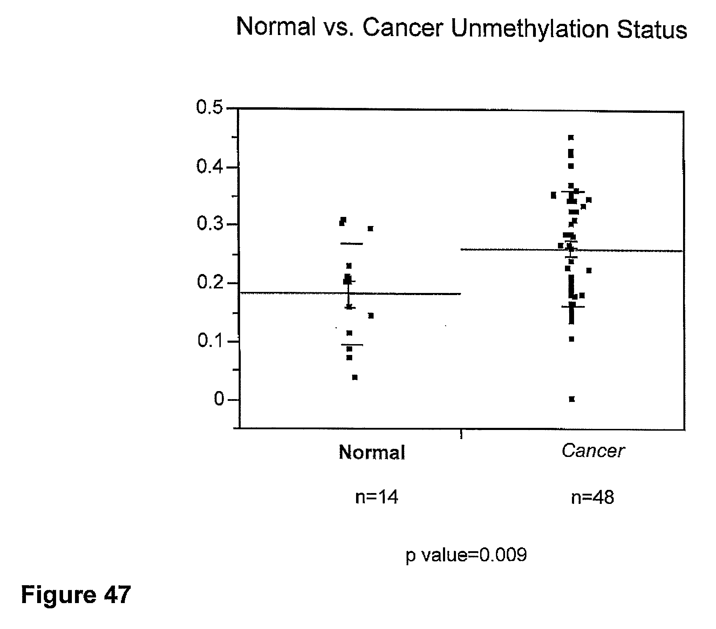

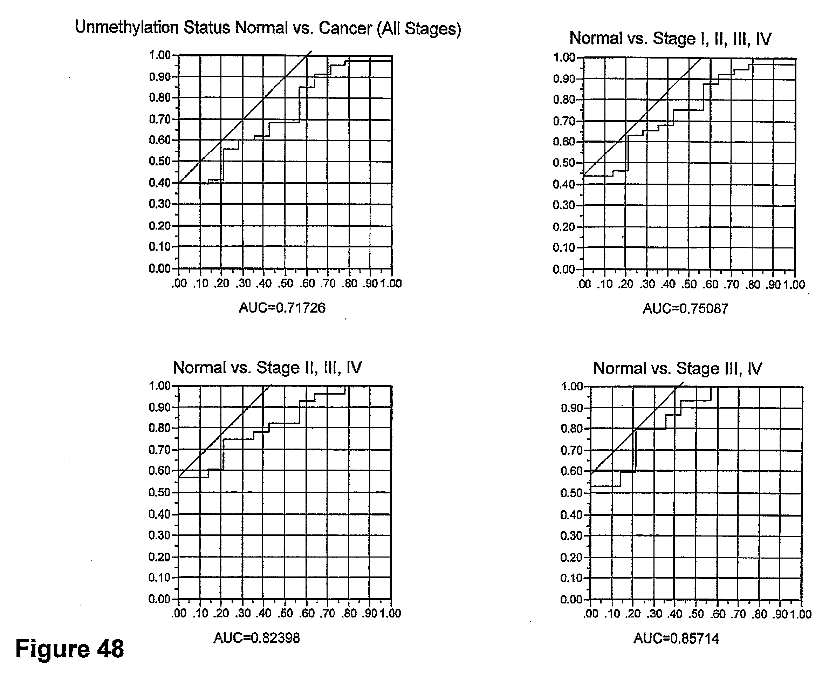

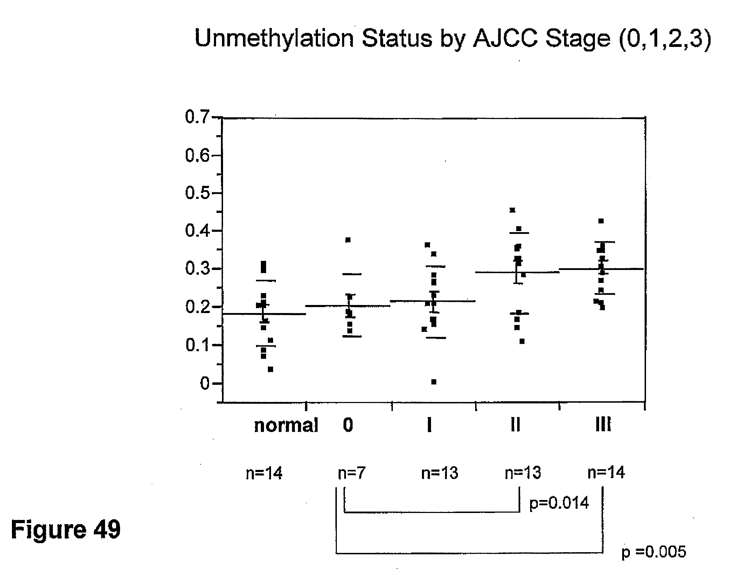

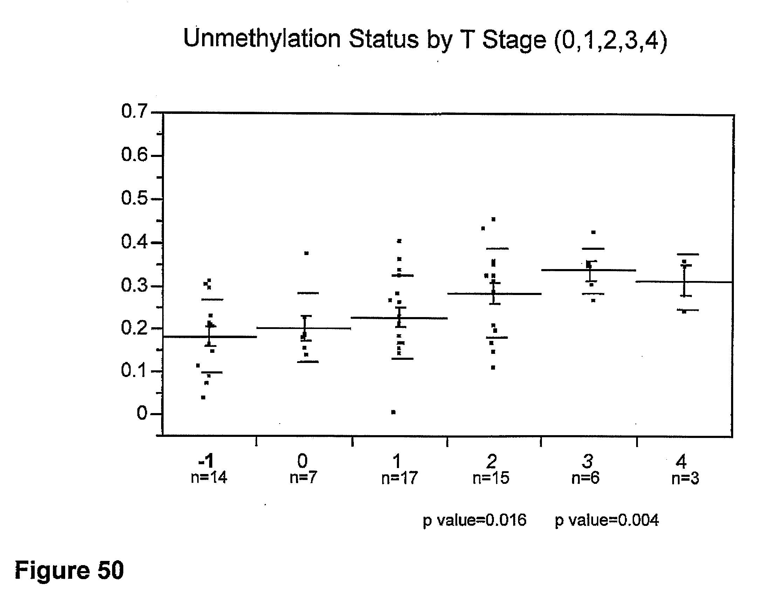

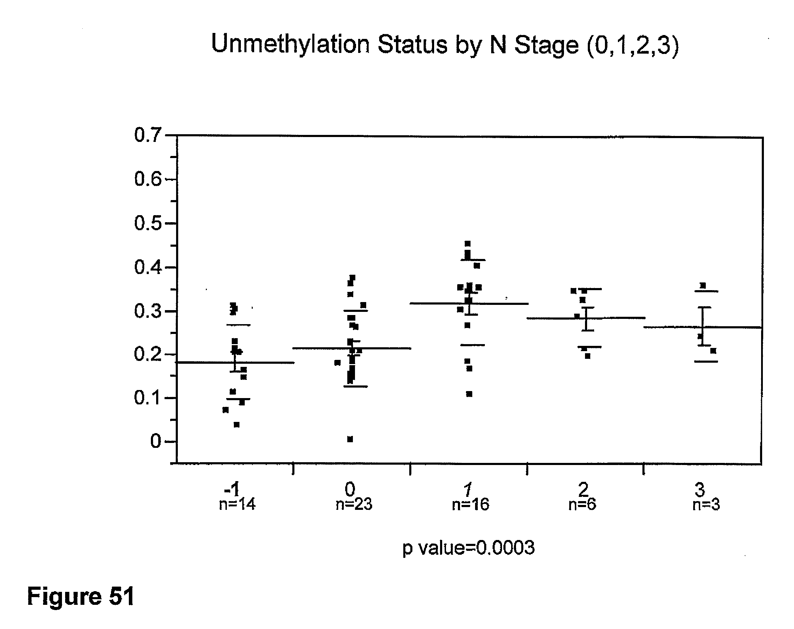

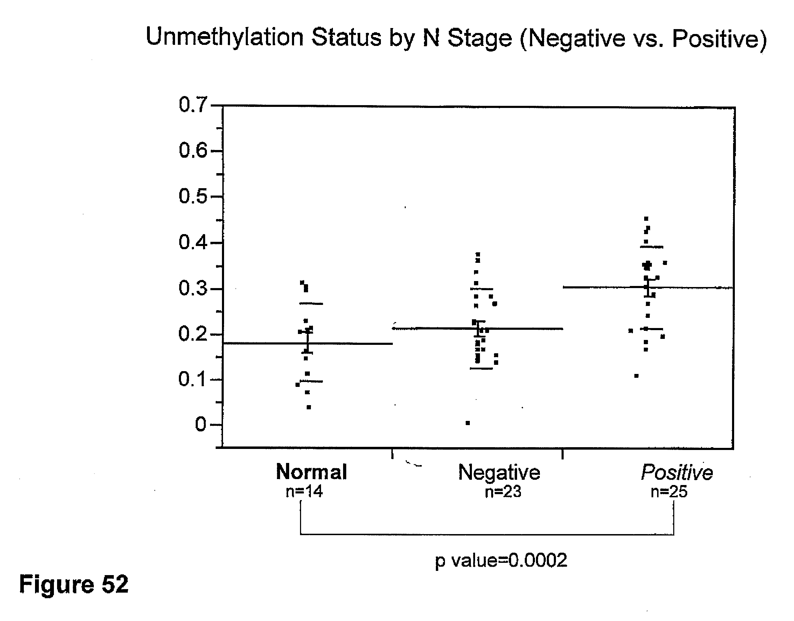

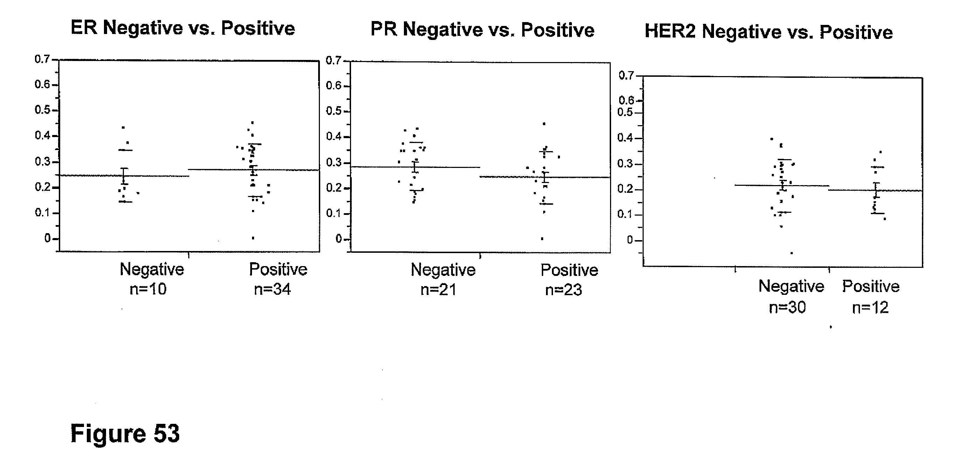

### E. HYPOMETHYLATION OF LINE-1 IN BREAST CANCER (FIGURES 37-53)

All publications cited herein are incorporated by reference in their entirety.

Preferably, a method of detecting LINE-1 (long interspersed nucleotide elements- 1) DNA in a body fluid can comprise providing a body fluid sample from a subject; and
detecting LINE-1 DNA in the sample.

Preferably, the LINE-1 DNA exists as cellular or acellular DNA in the subject.

Preferably, the method further comprises detecting methylation or unmethylation of the LINE- 1 DNA at the promoter region.

Preferably, the body fluid is blood, serum, plasma, bone marrow, peritoneal fluid, or cerebral spinal fluid.

Preferably, he subject suffers from cancer.

Preferably, the cancer is prostate cancer, esophageal cancer, colorectal cancer, melanoma, or breast cancer.

Preferably, a method determines whether a subject is suffering from cancer can comprise providing a body fluid sample from a subject; and determining the level of LINE-1 DNA in the sample, wherein the level of the LINE-1 DNA in the sample, if higher than a control LINE-1 level in a normal sample, indicates that the subject is likely to be suffering from cancer.

Preferably, the LINE-I DNA exists as cellular or acellular DNA in the subject.

Preferably, the level of the LINE-1 DNA is represented by the level of the LINE-1 DNA either methylated or unmethylated at the promoter region, the level of the LINE-1 DNA unmethylated at the promoter region, or the ratio of the level of the LINE-1 DNA unmethylated at the promoter region to the level of the LINE-1 DNA either methylated or unmethylated at the promoter region. Preferably, the body fluid is blood, serum, plasma, bone marrow, peritoneal fluid, or cerebral spinal fluid.

Preferably, the cancer is prostate cancer, esophageal cancer, colorectal cancer, melanoma, or breast cancer.

Preferably, a method determines whether a subject is suffering from cancer, can comprise providing from a subject a sample of a tissue where esophageal cancer, colorectal cancer, melanoma, or breast cancer may develop; and determining the level of LINE-I DNA in the sample, wherein the level of the LINE-I DNA in the sample, if higher than a control LINE-I level in a normal sample, indicates that the subject is likely to be suffering from esophageal cancer, colorectal cancer, melanoma, or breast cancer.

Preferably, the level of the LINE-I DNA is represented by the level of the LINE-I DNA either methylated or unmethylated at the promoter region, the level of the LINE-I DNA unmethylated at the promoter region, or the ratio of the level of the LINE-I DNA unmethylated at the promoter region to the level of the LINE-I DNA either methylated or unmethylated at the promoter region.

Preferably, a method of monitoring cancer can comprise providing a tumor or body fluid sample from a subject suffering from cancer; and determining the level of LINE-I DNA in the sample, wherein the level of the LINE-I DNA in the sample, if higher than a control LINE-I level in a control tumor or body fluid sample from a control subject suffering from the cancer, indicates that the cancer is likely to be at a more advanced stage in the subject than in the control subject, the subject is likely to be less responsive to a cancer therapy than the control subject, or the tumor genetic instability is likely to be higher in the subject than in the control subject; or the level of the LINE-I DNA in the sample, if lower than a control LINE-I level in a control tumor or body fluid sample from a control subject suffering from the cancer, indicates that the cancer is likely to be at a less advanced stage in the subject than in the control subject, the subject is likely to be more responsive to a cancer therapy than the control subject, or the tumor genetic instability is likely to be lower in the subject than in the control subject.
Preferably, the LINE-I DNA exists as cellular or acellular DNA in the subject.

Preferably, the level of the LINE-I DNA is represented by the level of the LINE-I DNA either methylated or unmethylated at the promoter region, the level of the LINE-I DNA unmethylated at the promoter region, or the ratio of the level of the LINE-I DNA unmethylated at the promoter region to the level of the LINE-I DNA either methylated or unmethylated at the promoter region.

Preferably, the body fluid is blood, serum, plasma, bone marrow, peritoneal fluid, or cerebral spinal fluid.

Preferably, the level of the LINE-I DNA in the sample, if higher than the control LINE-I level in the control tumor or body fluid sample from the control subject suffering from the cancer, indicates that the level of RASSFIa, RARb, or GSTP1 gene unmethylated at the promoter region is likely to be higher in the sample than in the control sample; or the level of the LINE-I DNA in the sample, if lower than the control LINE-I level in the control tumor or body fluid sample from the control subject suffering from the cancer, indicates that the level of RASSFIa, RARb, or GSTPI gene unmethylated at the promoter region is likely to be lower in the sample than in the control sample.

Preferably, the cancer is prostate cancer, esophageal cancer, colorectal cancer, melanoma, or breast cancer.

Preferably, the level of the LINE-I DNA in the sample, if higher than the control LINE-I level in the control tumor or body fluid sample from a control subject suffering from a multifocal prostate cancer, indicates that the subject is likely to be suffering from a unifocal prostate cancer; or the level of the LINE-I DNA in the sample, if lower than the control LINE-I level in the control tumor or body fluid sample from a control subject suffering from a unifocal prostate cancer, indicates that the subject is likely to be suffering from a multifocal prostate cancer; or wherein the level of the LINE-I DNA in the sample, if higher than the control LINE-I level in the control tumor or body fluid sample from the control subject suffering from the cancer, indicates that the prostate volume is likely to be larger in the subject than in the control subject, or the PSA density is likely to be higher in the subject than in the control subject; or the level of the LINE-I DNA in the sample, if lower than the control LINE-1 level in the control tumor or body fluid sample from the control subject suffering from the cancer, indicates that the prostate volume is likely to be smaller in the subject than in the control subject, or the PSA density is likely to be lower in the subject than in the control subject.

## Claims

1. A method of monitoring cancer, comprising:
determining the level of LINE-1 DNA in a body fluid sample from a subject suffering from cancer;
comparing the level of the LINE-1 DNA in the sample to a control LINE-1 level in a control body fluid sample from a control subject suffering from the cancer; and
determining that the cancer is likely to be at a more advanced stage in the subject than in the control subject, the subject is likely to be less responsive to a cancer therapy than the control subject, or the tumor genetic instability is likely to be higher in the subject than in the control subject when the level of the LINE-1 DNA in the sample is higher than the control LINE-1 level in the control body fluid sample.

2. The method of claim 1, further comprising determining that the cancer is likely to be at a less advanced stage in the subject than in the control subject, the subject is likely to be more responsive to a cancer therapy than the control subject, or the tumor genetic instability is likely to be lower in the subject than in the control subject when the level of the LINE-1 DNA in the sample is lower than the control LINE-1 level in the control body fluid sample.

3. The method of claim 1, wherein the LINE-1 DNA exists as acellular DNA in the subject.

4. The method of clam 1, wherein the level of the LINE-1 DNA is represented by
(i) the level of the LINE-1 DNA either methylated or unmethylated at the promoter region;
(ii) the level of the LINE-1 DNA unmethylated at the promoter region; or
(iii) the ratio of the level of the LINE-1 DNA unmethylated at the promoter region to the level of the LINE-1 DNA either methylated or unmethylated at the promoter region.

5. The method of clam 1, wherein the body fluid is blood, serum, plasma, bone marrow, peritoneal fluid, or cerebral spinal fluid.

6. The method of claim 1, wherein the level of the LINE-1 DNA in the sample, if higher than the control LINE-1 level in the control body fluid sample from the control subject suffering from the cancer, indicates that the level of RASSF1a, RARb, or GSTP1 gene unmethylated at the promoter region is likely to be higher in the sample than in the control sample.

7. The method of claim 1, wherein the level of the LINE-1 DNA in the sample, if lower than the control LINE-1 level in the control body fluid sample from the control subject suffering from the cancer, indicates that the level of RASSF1a, RARb, or GSTP1 gene unmethylated at the promoter region is likely to be lower in the sample than in the control sample.

8. The method of claim 1, wherein the cancer is prostate cancer, esophageal cancer, colorectal cancer, melanoma, or breast cancer.

9. The method of claim 6, wherein the level of the LINE-1 DNA in the sample, if higher than the control LINE-1 level in the control body fluid sample from a control subject suffering from a multifocal prostate cancer, indicates that the subject is likely to be suffering from a unifocal prostate cancer.

10. The method of claim 6, wherein the level of the LINE-1 DNA in the sample, if lower than the control LINE-1 level in the control body fluid sample from a control subject suffering from a unifocal prostate cancer, indicates that the subject is likely to be suffering from a multifocal prostate cancer.

11. The method of claim 6, wherein the level of the LINE-1 DNA in the sample, if higher than the control LINE-1 level in the control body fluid sample from the control subject suffering from the cancer, indicates that the prostate volume is likely to be larger in the subject than in the control subject, or the PSA density is likely to be higher in the subject than in the control subject.

12. The method of claim 6, wherein the level of the LINE-1 DNA in the sample, if lower than the control LINE-1 level in the control body fluid sample from the control subject suffering from the cancer, indicates that the prostate volume is likely to be smaller in the subject than in the control subject, or the PSA density is likely to be lower in the subject than in the control subject.

13. A method of monitoring cancer, comprising:
determining the level of LINE-1 DNA in a tumor or body fluid sample from a subject suffering from esophageal cancer, colorectal cancer, melanoma, or breast cancer, wherein the level of the LINE-1 DNA in the sample, if higher than a control LINE-1 level in a control tumor or body fluid sample from a control subject suffering from the esophageal cancer, colorectal cancer, melanoma, or breast cancer, indicates that the esophageal cancer, colorectal cancer, melanoma, or breast cancer is likely to be at a more advanced stage in the subject than in the control subject, the subject is likely to be less responsive to an esophageal cancer, colorectal cancer, melanoma, or breast cancer therapy than the control subject, or the tumor genetic instability is likely to be higher in the subject than in the control subject.

14. The method of claim 13, further comprising determining the level of LINE-1 DNA in a tumor or body fluid sample from a subject suffering from esophageal cancer, colorectal cancer, melanoma, or breast cancer, wherein the level of the LINE-1 DNA in the sample, if lower than a control LINE-1 level in a control tumor or body fluid sample from a control subject suffering from the esophageal cancer, colorectal cancer, melanoma, or breast cancer, indicates that the esophageal cancer, colorectal cancer, melanoma, or breast cancer is likely to be at a less advanced stage in the subject than in the control subject, the subject is likely to be more responsive to an esophageal cancer, colorectal cancer, melanoma, or breast cancer therapy than the control subject, or the tumor genetic instability is likely to be lower in the subject than in the control subject.

15. A method of monitoring cancer, comprising:
determining the level of LINE-1 DNA in a tumor or body fluid sample from a subject suffering from prostate cancer, wherein the level of the LINE-1 DNA in the sample, if higher than a control LINE-1 level in a control tumor or body fluid sample from a control subject suffering from the prostate cancer, indicates that the subject is likely to be less responsive to a prostate cancer therapy than the control subject; or the level of the LINE-1 DNA in the sample, if lower than a control LINE-1 level in a control tumor or body fluid sample from a control subject suffering from the prostate cancer, indicates that the subject is likely to be more responsive to a prostate cancer therapy than the control subject.
